(19) Europäisches Patentamt
European Patent Office
Office européen des brevets

(11) EP 4 241 792 A1

(12) EUROPEAN PATENT APPLICATION

(43) Date of publication:
**13.09.2023 Bulletin 2023/37**

(21) Application number: **22160879.7**

(22) Date of filing: **08.03.2022**

(51) International Patent Classification (IPC):
***A61K 49/12*** *(2006.01)* ***A61K 49/18*** *(2006.01)*

(52) Cooperative Patent Classification (CPC):
**A61K 49/12; A61K 49/18**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Spago Nanomedical AB**
**223 63 Lund (SE)**

(72) Inventors:
- **AXELSSON, Oskar**
**243 32 Höör (SE)**
- **EKENGARD, Erik**
**212 31 Malmö (SE)**
- **LIU, Yi-Chi**
**224 71 Lund (SE)**
- **PARIS, Juraj**
**211 28 Malmö (SE)**

(74) Representative: **Ström & Gulliksson AB**
**P.O. Box 793**
**220 07 Lund (SE)**

(54) **NANOSTRUCTURES AND APPLICATIONS THEREOF**

(57) The present disclosure relates to a plurality of globular nanostructures, having a dispersity between 1 and 1.8 and a volume average hydrodynamic diameter of 13 nm to 90 nm; wherein each nanostructure comprises a polymer framework of monomer residues, wherein the average number of bonds from each monomer residue is in the range of from 3.0 up to but not including 6.0; wherein at least 90% of the monomer residues comprise two geminal chelating groups, each chelating group independently being -PO($OR^1$)($OR^2$); wherein $R^1$ and $R^2$ are independently selected from the group consisting of a negative charge and H; and - denotes an internal bond in the monomer residue. The present disclosure also relates to a method of producing such nanostructures, to the use of such nanostructures as well as to a pharmaceutical composition comprising such nanostructures.

Fig. 2

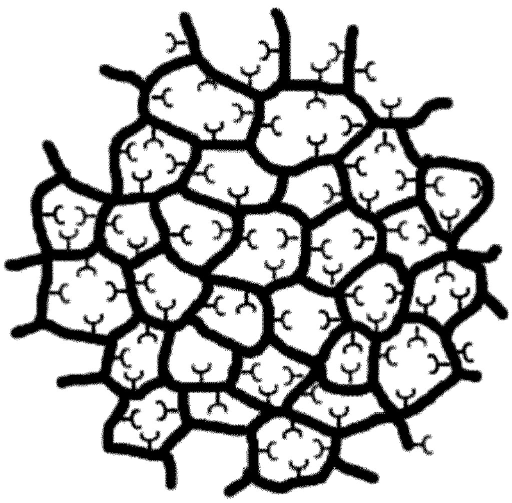

EP 4 241 792 A1

## Description

### TECHNICAL FIELD

The present disclosure relates to chelating polymeric nanostructures with applications as intermediates in the production of nanostructures with applications in systemic radiotherapy and cancer imaging.

### BACKGROUND

The gold standard for cancer treatment is surgery. In cases where surgery alone is not curative, multimodality regimens including chemotherapy and radiation treatment are used. About half of all cancer patients today are treated with radiotherapy, either alone or in combination with other treatments. Radiation delivered as external beams offers a relatively simple and practical approach to causing radiation damage to the tumor. Although the intensity, location and timing for external radiation can be well controlled and modulated, disadvantages associated with this technique include the destruction of normal tissue in the path of the beam as well as damage to tissues surrounding the tumor. The risk of damaging surrounding healthy tissue speaks against external radiotherapy to deeply situated tumors and tumors situated next to vital organs. Furthermore, high radiation doses are frequently required to penetrate the tissue. Moreover, in order to be efficient, external radiotherapy often requires the patients to submit themselves to daily hospital visits over extended periods of time.

Systemic radiotherapy, which internally delivers radioactive substances to the tumor, offers solutions to many of the above-mentioned disadvantages connected with external radiotherapy.

The most commonly used radionuclides for systemic radiotherapy in clinics today are beta-emitters. The maximum tissue penetration range (1-10 mm) and cross-fire effects, i.e. the ability to kill cells indirectly along a longer path length, of beta-particles with energies between 0.1-2.2 MeV allows for the targeting of tumor cells in close proximity to neovasculature. Radionuclides such as $^{131}I$ are used alone, or conjugated with monoclonal antibodies or peptides to allow for tumor-targeting radioimmunotherapy. Clinical use of alphaemitters is less common, but some show clinical potential, e.g. $^{223}Ra$.

Recent advances in nanotechnology have led to the development of novel nanocarriers designed for cancer detection and screening, *in vivo* molecular and cellular imaging as well as the delivery of therapeutics. However, despite a large number of publications covering nanosized carriers for cancer therapeutics, relatively few have reached clinical trials, and only a handful are approved by the FDA.

The rationale for nanosized materials being suitable as tumor-targeting radiation carriers is related to the enhanced permeation and retention (EPR) effect. The EPR effect is based on the fact that whereas the capillaries of healthy tissues are virtually impermeable to molecules larger than 3-4 nm, capillaries of fast-growing tumor tissue are much leakier. In addition, solid tumors tend to lack functional lymphatics. Combined, these features limit the removal of extravasated nanomaterials from most solid tumors, leading to an accumulation of nanosized materials in the tumor. Because EPR-mediated drug targeting exclusively relies on the pathological properties of the target tissue, i.e. enhanced leakiness and poor lymphatic drainage, it is generally referred to as passive tumor targeting.

EP2923712A1 discloses nanostructures, having a hydrodynamic diameter of 8-100 nm, that can be used in imaging and/or radiotherapy.

US20140004048 describes a nanoparticle that is dendrimized to enhance the solubility of the nanoparticle.

Many of the approaches to radioisotope therapy involving alleged nanocarriers suffer from the drawback that the nanocarrier is not really a nanocarrier as it is larger than 100 nm and due to its large size suffers from the drawback of not delivering the radioisotope to the tumor tissue in an effective way. WO 2004/040972 is one example of a carrier that is larger than 100 nm.

### SUMMARY

According to a first aspect, the above and other objects are achieved, in full or at least in part by a composition as defined by claim 1. According to this claim, the above object is achieved by a plurality of globular nanostructures, wherein the plurality of globular nanostructures has a dispersity between 1 and 1.8; and wherein the nanostructures have a volume average hydrodynamic diameter of 13 nm to 90 nm and wherein each nanostructure comprises a polymer framework of monomer residues, wherein the average number of bonds from each monomer residue is in the range of from 3.0 up to but not including 6.0; wherein the linkages between the monomer residues are Si-O-Si; wherein each nanostructure comprises from 10% to 25% by weight of silicon; wherein at least 90% of the monomer residues have from 5 to 11 carbon atoms; wherein at least 90% of the monomer residues comprise two geminal chelating groups, each chelating group independently being a group according to Formula (I)

$$-PO(OR^1)(OR^2) \qquad (I)$$

wherein $R^1$ and $R^2$ are independently selected from the group consisting of a negative charge and H; and - denotes an internal bond in the monomer residue; and wherein the chelating groups according to Formula (I) constitute at least 90% of the chelating groups in the nanostructure.

The term "two geminal chelating groups" refers to two chelating groups separated by one carbon atom, i.e. the two chelating groups are bound to the same carbon atom.

Importantly, the present disclosure relates to a plurality of globular nanostructures with a defined dispersity. Further, the globular nanostructures described herein can be used as the "core" or "central part" of a coated nanoparticle. A plurality of such coated nanostructures will also have a low dispersity. Several advantages, some of which are newly discovered, are associated with a low dispersity. As shown in example 6, the optimum size for delivery to tumors is around 30 nm. Thus, the lower the dispersity of said coated nanostructures, the better the delivery to tumors, as long as the average diameter is around 30 nm. Since the dispersity of the of the core nanostructures translates to dispersity of coated nanostructures it is advantageous that a plurality of nanostructures has a low dispersity. Also, low dispersity gives less losses during production of coated nanostructures and thus gives better economy since coated nanostructures outside the desired range will otherwise have to be filtered off.

As mentioned above, the plurality of globular nanostructures has a dispersity of between 1 and 1.8. The dispersity can be measured by DLS as specified below or by other methods known in the art. Nanostructures having such a low dispersity are especially useful for producing coated nanostructures that can be used in in vivo applications, such as imaging and radiotherapy.

The dispersity may be between 1 and 1.5, between 1 and 1.3, or between 1.1 to 1.35, such as less than 1.3.

The dispersity of the of the core nanostructures of the plurality of nanostructures according to the present disclosure translates to dispersity of coated nanostructures (described below). Notably, the lower the dispersity of said coated nanostructures, the better the delivery to tumors.

The nanostructures may have a volume average hydrodynamic diameter of from 13 to 90 nm, such as from 13 to 50 nm, such as from 13 to 30 nm, such as from 14 to 25 nm, such as from 15 to 22 nm, such as from 16 to 20 nm. The volume average hydrodynamic diameter may thus be from 17 to 19 nm, such as 18 nm.

The nanostructures according to the present disclosure are small enough to be able to leak out through defective capillaries and diffuse through the intracellular matrix and deliver the radioactivity to the tumor cells.

The "average number of bonds from each monomer residue" is the average number of bonds from the monomers to the polymeric framework, i.e. to other monomers.

The polymer network may be amorphous.

The polymeric framework may be a homopolymer of a single monomer or a copolymer of two or more different monomers, preferably with a randomly branching and cross-linking pattern. This is in contrast to e.g. the orderly branching pattern in dendrimers. In the case of the polymeric framework being a copolymer, the nanostructures comprise random order polymers.

The average number of bonds from each monomer residue may be in the range of from 3.5 to 5.9, such as from 3.7 to 5.5, such as from 4.0 to 5.0, such as 4.5. A higher number of bonds from each monomer residue often gives an increased storage- and *in vivo*-stability. Furthermore, higher crosslinking gives a denser nanostructure, in which the chelating phosphonate groups ($-PO(OR^1)(OR^2)$) are located closer to each other. This is considered to give a higher chelate stability.

As mentioned above, the nanostructure comprises from 10% to 25% by weight of silicon. The weight of silicon refers to the percentage of silicon in a dry sample of nanostructures. A drying procedure is found in example 11 below. In some examples, the nanostructure comprises from 13% to 18% by weight of silicon.

As mentioned above, at least 90% of the monomer residues have from 5 to 11 carbon atoms. Preferably, at least 95%, such as at least 99%, of the monomer residues have from 7 to 9 carbon atoms.

As mentioned above, at least 90% of the monomer residues comprise two geminal chelating groups according to Formula (I) as described above. Preferably, at least 92%, such as at least 95%, such as at least 97%, such as at least 99%, such as 100% of the monomer residues comprise two chelating groups according to Formula (I) as described above.

The chelating groups according to Formula (I) may make up at least 95%, such as at least 99%, of the chelating groups in the nanostructure.

The globular, chelating polymeric nanostructures according to the present disclosure can function as intermediates in the production of coated nanostructures with applications in radioisotope therapy and cancer diagnostics. In particular, they are suitable to serve as the central part in coated nanostructures with applications in systemic radiotherapy and cancer imaging.

The nanostructures according to the present disclosure are bioinert, as biodegradability would cause undesirable and uncontrolled loss of the radioactive isotope from the nanostructure. Such uncontrolled loss of the radioactive isotope may cause radiation damage in vital organs, such as the liver and kidneys.

The nanostructures according to the present disclosure may function as intermediates for the production of nanocarriers or nanomaterials that are above the threshold where they would be excreted through the kidneys and thereby either cause damage and/or be lost from the body. At the same time, the nanostructures and coated nanostructures according to the present disclosure are small enough (having a diameter of below 90 nm and below 100 nm, respectively) to be able to leak out through defective capillaries and diffuse through the intracellular matrix and deliver the radioactivity to the tumor cells. Although in no way certain or limiting, it is thus conceivable that the EPR effect is often the basis of the favorable tumor delivery properties of the materials derived from the nanostructures according to the present disclosure.

According to one embodiment, at least 90% of the monomer residues, as incorporated into the polymeric framework, are residues according to Formula (II) :

$$\{(OR^1)(OR^2)PO\}_2\text{-}(C)\{(CH_2)_nSi(OR^3)_3\}\{(CH_2)_nSi(OR^3)_3\} \qquad \text{(II)}$$

wherein each $R^1$ and $R^2$ is independently selected from the group consisting of a negative charge and H; each $R^3$ is independently selected from the group consisting of a negative charge, H and a covalent bond to the polymeric framework; wherein at least 3 $R^3$ are bonds to the polymeric framework; and n is an integer between 1 and 5.

Since the crosslinking between the monomers is higher in a nanostructure according to the present disclosure in which at least 3 $R^3$ are bonds to the polymeric framework than in a corresponding nanostructure in which less than 3 $R^3$ are bonds to the polymeric framework, such a nanostructure has a better storage- and *in vivo*-stability. Furthermore, higher crosslinking gives a denser nanostructure, in which the chelating phosphonate groups ($-PO(OR^1)(OR^2)$) are located closer to each other. This is considered to give a higher chelate stability.

At least 4, such as at least 5, such as 6, of the $R^3$-groups may represent a bond to the polymeric framework.

At least 4 of the $R^3$-groups may be bonds to the polymeric framework.

In some examples, at least 5, such as 6 of the $R^3$-groups may be bonds to the polymeric framework.

In one embodiment, n = 3. Such nanoparticles have been demonstrated to be especially efficient.

In one specific embodiment, at least 3 $R^3$-groups are bonds to the polymeric framework; and n = 3.

In one specific embodiment, at least 4 $R^3$-groups are bonds to the polymeric framework; and n = 3.

In one specific embodiment, at least 5 $R^3$-groups are bonds to the polymeric framework; and n = 3.

In one specific embodiment, all 6 $R^3$-groups are bonds to the polymeric framework; and n = 3.

The nanostructures may further comprise a coating. Such a coating contributes to the biocompatibility of the nanostructures.

Typically, a coated nanostructure as described herein has a volume average hydrodynamic diameter of 18-100 nm, such as 20-50 nm, such as 25-35 nm.

Preferably, the coating adds at least 5 nm, such as 5 to 25 nm, such as 10 to 20 nm, such as 15 nm, to the diameter of the nanoparticle. In other words, the thickness of the coating is at least 2.5 nm, such as 2.5 to 12.5 nm, such as 5 - 10 nm, such as 7.5 nm.

The coating contributes to the biocompatibility of the nanostructures.

Preferably, the coating comprises hydrophilic groups. This confers water solubility, biocompatibility and stability to aggregation.

Thus, the coating may be a coating of hydrophilic polymers, such as a coating comprising polyethylene glycol (PEG), polyoxazoline, or peptoids. Preferably, each polyethylene glycol chain comprises between 10 and 150 ethylene glycol units, such as between 20 and 100, such as between 30 and 50 ethylene glycol residues, such as 45 ethylene glycol residues.

The coating may comprise coating monomer residues comprising one or multiple polyethylene glycol chains and one or multiple silicon atoms, joined through an organic linker.

Preferably, the coating comprises coating monomer residues comprising 2 silicon atoms and 2 polyethylene glycol chains, each polyethylene glycol chain comprising between 20 and 100 ethylene glycol units.

Specifically, the coating may comprise coating monomer residues comprising one or multiple polyethylene glycol chains and one or multiple silicon atoms, joined through an organic linker. The organic linker may be a hydrocarbon. The organic linker may also comprise ether bonds. The silicon atoms in the monomer may be present in reactive siloxane groups, such as - $Si(OEt)_3$, $-Si(OMe)_3$, or $-SiCl_3$.

When the coating comprises coating monomer residues comprising silicon atoms, the coating monomer residues are bound to the rest of the nanostructure through siloxane bonds.

The coating may be grafted onto the nanostructures through a reaction between the nanostructure and coating monomers comprising reactive siloxane groups, such as $-Si(OEt)_3$, $-Si(OMe)_3$, or $-SiCl_3$.

Preferred coating monomers are $(m\text{-}PEG_xOCH_2)_2C]\text{-}CH_2CH_2CH_2\text{-}Si(OEt)_3]$, wherein m is short for methyl and x is an integer between 10 and 150, such as between 20 and 100, such as between 30 and 50, such as 45 ethylene glycol residues.

Other preferred coating monomers are m-$PEG_x$-$CH_2CH_2CH_2$-$Si(OEt)_3$, wherein m is short for methyl and x is an integer between 10 and 150, such as between 20 and 100, such as between 30 and 50, such as 45 ethylene glycol residues.

Other preferred coating monomers are 1-(ω-methyl-(ethyleneoxy)$_x$methyl)-3,5-bis(3-(triethoxysilyl)propyloxy)-benzene, wherein x is an integer between 10 and 150, such as between 20 and 100, such as between 30 and 50, such as 45; and 1,7-bis(triethoxysilyl)-4,4-bis(ω-methyl-(ethyleneoxy)$_x$methyl)-heptane wherein x is an integer between 10 and 150, such as between 20 and 100, such as between 30 and 50, such as 45.

According to a second aspect, there is disclosed a pharmaceutical composition comprising a plurality of globular nanostructures comprising a coating according to the present disclosure. Preferably, the hydrophilic groups of the coating are as described above.

The pharmaceutical composition may be in the form of a liquid solution. Preferably, the liquid solution is an aqueous solution. In one embodiment, the aqueous solution has a pH below 3.5.

The pharmaceutical composition may further comprise a liquid, such as water.

The pharmaceutical composition may further comprise at least one antioxidant and/or at least one cryoprotectant.

The globular nanostructures of the pharmaceutical composition may further comprise radioactive isotope(s). Suitable radioactive isotope(s) are described below.

The pharmaceutical composition according to the second aspect may be a pharmaceutical composition for use as a medicament.

According to a third aspect, there is provided a pharmaceutical composition for use in the treatment of cancer and/or in imaging, wherein the pharmaceutical composition comprises a plurality of globular nanostructures comprising a coating according to the present disclosure, wherein the globular nanostructures further comprise radioactive isotope(s). Preferably, the hydrophilic groups of the coating are as described above.

The imaging may be PET-imaging or SPECT-imaging.

The radioactive isotope may be chosen from the group consisting of actinium-225 ($^{225}Ac$); copper-62 ($^{62}Cu$); copper-64 ($^{64}Cu$); copper-67 ($^{67}Cu$); gallium-67 ($^{67}Ga$); gallium-68 ($^{68}Ga$); holmium-166 ($^{166}Ho$); indium-111 ($^{111}In$); lead-212 ($^{212}Pb$); lutetium-177 ($^{177}Lu$); radium-223 ($^{223}$ Ra); rhenium-186 ($^{186}Re$); rhenium-188 ($^{188}Re$); rubidium-82 ($^{82}Rb$); samarium-153 ($^{153}Sm$); strontium-89 ($^{89}Sr$); technetium-99m ($^{99m}Tc^{3+}$); thallium-201 ($^{201}Tl$); thorium-227 ($^{227}Th$); yttrium-86 ($^{86}Y$); yttrium-90 ($^{99}Y$); and zirconium-89 ($^{89}Zr$), and combinations thereof. Thus, two or more radioactive isotopes may be present in the pharmaceutical composition for use in imaging and/or treatment of cancer.

Specifically, the radioactive isotope for therapy may be chosen from the group consisting of actinium-225 ($^{225}Ac$); copper-64 ($^{64}Cu$); copper-67 ($^{67}Cu$); holmium-166 ($^{166}Ho$); lead-212 ($^{212}Pb$); lutetium-177 ($^{177}Lu$); radium-223 ($^{223}Ra$); rhenium-186 ($^{186}Re$); rhenium-188 ($^{188}Re$); samarium-153 ($^{153}Sm$); strontium-89 ($^{89}Sr$); thorium-227 ($^{227}Th$) and yttrium-90 ($^{90}Y$) and combinations thereof. Thus, two or more radioactive isotopes may be present in the pharmaceutical composition for use in treatment of cancer.

Specifically, the radioactive isotope for imaging may be chosen from the group consisting of copper-62 ($^{62}Cu$); copper-67 ($^{67}Cu$); gallium-67 ($^{67}Ga$); gallium-68 ($^{68}Ga$); indium-111 ($^{111}In$); lutetium-177 ($^{177}Lu$); rhenium-186 ($^{186}Re$); rubidium-82 ($^{82}Rb$): technetium-99m ($^{99m}Tc^{3+}$); Thallium-201 ($^{201}Tl$); yttrium-86 ($^{86}Y$) and zirconium-89 ($^{89}Zr$), and combinations thereof. Thus, two or more radioactive isotopes may be present in the pharmaceutical composition for use in imaging.

Radioactive isotopes suitable for PET imaging may be chosen from the group consisting of copper-62 ($^{62}Cu$); gallium-68 ($^{68}Ga$); rubidium-82 ($^{82}Rb$); yttrium-86 ($^{86}Y$), and zirconium-89 ($^{89}Zr$), and combinations thereof. Thus, two or more radioactive isotopes may be present in the pharmaceutical composition for use in PET-imaging.

Radioactive isotopes suitable for SPECT-imaging may be chosen from the group consisting of gallium-67 ($^{67}Ga$); indium-111 ($^{111}In$); technetium-99m ($^{99m}Tc^{3+}$); lutetium-177 ($^{177}Lu$), and thallium-201 ($^{201}Tl$), and combinations thereof. Thus, two or more radioactive isotopes may be present in the pharmaceutical composition for use in SPECT-imaging.

In one specific embodiment of the pharmaceutical composition, the nanostructures comprise a coating as described above and at least 90% of the monomer residues of the central part of the nanostructure are residues according to Formula (II):

$$\{(OR^1)(OR^2)PO\}_2\text{-}(C)\{(CH_2)_nSi(OR^3)_3\}\{(CH_2)_nSi(OR^3)_3\} \qquad (II)$$

wherein each $R^1$ and $R^2$ is independently selected from the group consisting of a negative charge and H; each $R^3$ is independently selected from the group consisting of a negative charge, H and a covalent bond to the polymeric framework; wherein at least 3 $R^3$ are bonds to the polymeric framework; and n is 3. Specifically, at least 4 of the $R^3$-groups are bonds to the polymeric framework.

Preferably, the coating comprises coating monomer residues comprising 2 silicon atoms and 2 polyethylene

glycol chains, each polyethylene glycol chain comprising between 20 and 100 ethylene glycol units.

According to a fourth aspect, there is provided a method for purifying 1,7-bis(triethoxysilyl)-4,4-bis(dimethoxyphosphonato)heptane, the method comprising the steps of (a) providing a solution of impure 1,7-bis(triethoxysilyl)-4,4-bis(dimethoxyphosphonato)heptane in a polar aprotic solvent; (b) separating the solution of step (a) from insoluble matter; (c) concentrating the solution obtained in step (b), thereby providing a residue; (d) dissolving the residue obtained in step (c) in a non-polar solvent; (e) separating the solution obtained in step (d) from insoluble matter; (f) removing water from the solution obtained in step (e); (g) concentrating the dried solution obtained in step (f), resulting in a second residue; (h) subjecting the residue obtained in step (g) to a short path, pass-through vacuum distillation; and (i) collecting the pass-through fraction from step (h), comprising purified 1,7-bis(triethoxysilyl)-4,4-bis(dimethoxyphosphonato)heptane.

The steps may be performed in the order (a), (b), (c), (d), (e), (f), (g), (h), (i).

The precipitation steps (steps (a) and (d)) are advantageous to remove oligomeric materials which may otherwise clog up the distillation setup.

The aprotic solvent in step (a) may be a nitrile, a ketone, an ester or a polar ether.

Examples of suitable nitriles are acetonitrile and propionitrile.

Examples of suitable ketones are acetone and methylethylketone.

Examples of suitable esters are ethyl actetate and isopropyl acetate.

Examples of suitable polar ethers are THF (terahydrofuran) and Me-THF (methyl-tetrahydrofuran).

Specifically, the aprotic solvent in step (a) may be acetonitrile. The impure 1,7-bis(triethoxysilyl)-4,4-bis(dimethoxyphosphonato)heptane may be dissolved in acetonitrile at a concentration between 5% (w/v) and 20% (w/v), such as between 7% (w/v) and 15% (w/v), such as 10% (w/v), (w/v) denoting weight/volume.

Step (b) may be performed by filtration.

Alternatively, step (b) may be performed by sedimentation and decantation and by filtration

Step (c) and/or step (g) may be performed by evaporation of the solvent.

The non-polar solvent in step (d) may be a cyclic or non-cyclic hydrocarbon or a mixture of hydrocarbons.

Examples of suitable cyclic hydrocarbons are cyclohexane and cycloheptane.

Examples of suitable non-cyclic hydrocarbons are pentane, hexane and heptane.

The non-polar solvent in step (d) may be heptane. The residue obtained in step (c) may be dissolved in heptane at a concentration such as between 5% (w/v) and 20% (w/v), such as between 7% (w/v) and 15% (w/v), such as 10% (w/v), (w/v) denoting weight/volume).

Step (e) may be performed by filtration.

Alternatively, step (e) may be performed by sedimentation and decantation.

Step (f) may be performed by drying the solution obtained in step (e) over a drying agent, e.g. using molecular sieves, such as 4Å molecular sieves. In other words, step (f) may be performed by drying the solution obtained in step (e) over activated molecular sieves, optionally followed by filtration. The solvent may be removed by evaporation. If molecular sieves are used in step (f), step (g) may be performed by separating the solution from the molecular sieves followed by evaporation of the solvent.

The step of the short path, pass-through vacuum distillation (h) may include setting the temperature of the heating element to a temperature from 150 °C to 190 °C, such as 160 °C to 180 °C, or such as 165 °C to 175 °C, and pass the crude product through the equipment. Then the lower boiling impurities of the character of lacking some structural feature, such as a silyl group, may be removed and collected as distillate and the purified product is retained.

Step (h) may be performed multiple times until a satisfactory purity has been achieved.

In an optional, second step of the short path, pass-through vacuum distillation, the heating element is set to a higher temperature such as from 170 °C to 190 °C and the once purified product is passed through the equipment once more. Then the product is vaporized and collected as distillate and non-volatiles are retained. This second step is advantageous since the production of many silanes, such as 1,7-bis-(triethoxysilyl)-4,4-bis-(dimethylphosphonato)-heptane, may involve a platinum catalyzed hydrosilylation step and may contain unacceptable amounts of platinum residues. Those are non-volatile and are removed by the second step.

In one embodiment of the method, the polar aprotic solvent in step (a) is acetonitrile and the solution in step (a) has a concentration of impure 1,7-bis(triethoxysilyl)-4,4-bis(dimethoxyphosphonato)heptane ranging from 25 g/l to 250 g/l; and/or the non-polar solvent in step (d) is a lower alkane, and the solution in step (d) has a concentration of the residue obtained in step (c) ranging from 25 g/l to 250 g/l/l; and/or the short path, pass-through vacuum distillation in step (h) is performed at a temperature ranging from 150 °C to 190 °C and a pressure ranging from 0.1 mbar to 1 mbar.

Preferably, the solution in step (a) has a concentration of impure 1,7-bis(triethoxysilyl)-4,4-bis(dimethoxyphosphonato)heptane ranging from 50 g/l to 200 g/l, more preferably 65 g/l to 150 g/l, even more preferably 75 g/l to 125 g/l, and most preferred 100 g/l.

Preferably, the non-polar solvent in step (d) is heptane.

Preferably, the solution in step (d) has a concentration of the residue obtained in step (c) ranging from 50 g/l

to 200 g/l, more preferably 65 g/l to 150 g/l, even more preferably 75 g/l to 125 g/l, and most preferred 100 g/l.

Preferably, the short path, pass-through vacuum distillation in step (h) is performed at a temperature ranging from 150 °C to 190 °C, preferably 160 °C to 180 °C, and a pressure ranging from 0.1 mbar to 1 mbar.

In a specific embodiment of the method, the polar aprotic solvent in step (a) is acetonitrile and the solution in step (a) has a concentration of impure 1,7-bis(triethoxysilyl)-4,4-bis(dimethoxyphosphonato)heptane ranging from 75 g/l to 125 g/l; and/or the non-polar solvent in step (d) is heptane and the solution in step (d) has a concentration of the residue obtained in step (c) ranging from 75 g/l to 125 g/l; and/or the short path, pass-through vacuum distillation in step (h) is performed at a temperature ranging from 160 °C to 180 °C, and a pressure ranging from 0.1 mbar to 1 mbar.

According to a fifth aspect, there is provided the use of 1,7-bis(triethoxysilyl)-4,4-bis(dimethoxyphosphonato)heptane purified according to the method according to the present disclosure to produce a plurality of globular nanostructures according to the present disclosure.

According to a sixth aspect, there is provided a method for producing a plurality of globular nanostructures according to the present disclosure, comprising the steps of (a) providing a solution comprising monomers in a mixture of water and a lower alcohol, wherein the monomers are monomers according to Formula (II)

$$\{(OR^1)(OR^2)PO\}_2\text{-}(C)\{(CH_2)_nSi(OR^3)_s\}\{(CH_2)_nSi(OR^3)_3\} \qquad (II)$$

wherein each $R^1$ and $R^2$ is independently selected from the group consisting of lower alkyls and aryl; and each $R^3$ is independently selected from the group consisting of lower alkyls and aryl; and n is an integer between 1 and 5; and (b) subjecting the solution of step (a) to a temperature between 110 and 160 °C.

Preferably, $R^1$ and $R^2$ are lower alkyls, such as comprising 1-8 carbon atoms. More preferably, $R^1$ and $R^2$ are $-CH_3$.

Preferably, the $R^3$-groups are lower alkyls, such as comprising 1-8 carbon atoms. More preferably, the $R^3$-groups are $-CH_2-CH_3$.

Preferably, n=3.

In a specifically preferred embodiment, $R^1$ and $R^2$ are $-CH_3$, the $R^3$groups are $-CH_2-CH_3$, and n=3.

Preferably, in step (b), the solution of step (a) is subjected to a temperature between 110 and 160 °C for a period of time such that rate of growth of the nanostructures is significantly lower than the initial rate of growth, for example to a temperature of 125 °C for a period of 200 hour, or to a temperature of 140 °C for a period of 48 hours.

Optionally, the method further comprises a step of (c) allowing the solution of step (b) to cool to ambient temperature.

Optionally, the method comprises a first step of purifying the monomers according to the method according to the present disclosure.

Preferably, the monomer has a purity of more than 80%, more preferably of more than 85%, more preferably more than 90%, and even more preferably more than 95%, especially more than 96%. In such cases, the size of the produced nanostructures plateaus at a given size. When this plateau has been reached, the degree of crosslinking is as high as it can get and the phosphonate esters are essentially completely hydrolyzed or hydrolyzed to a large extent. Additionally, using a monomer of such purity gives a more narrow size distribution of the produced nanostructures.

The monomer may be 1,7-bis(triethoxysilyl)-4,4-bis(dimethoxyphosphonato)heptane, preferably purified according to the method according to the present disclosure.

The mixture of water and a lower alcohol may be a mixture of 5 to 50% (vol/vol), preferably 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol) ((vol/vol) denoting volume/volume), of water in a lower alcohol, the lower alcohol having 1-8 carbon atoms.

Preferably, the lower alcohol is chosen from the group consisting of ethanol, 1- propanol, 2-propanol, or 1,2-propanediol, 1,3-propanediol and ethylene glycol or mixtures thereof.

A suitable solvent mixture is water/ethylene glycol in a suitable ratio such as from 50% to 95% ethylene glycol or from 80% to 95% ethylene glycol or from 88 to 92% ethylene glycol, i.e. 5-50% (vol/vol), such as 5-20% (vol/vol), such as 8-12% (vol/vol) of water in ethylene glycol.

The heating can occur either in a sealed, pressure resistant vessel or as reflux at atmospheric pressure.

The temperature in step (b) may be 115 to 145 °C, such as 120 to 145 °C, such as 125 to 145 °C, such as 130 to 145 °C, such as 135 to 145 °C, or such as 140 °C.

The minimum time period in step (b) is dependent on the temperature, such that a temperature of 125 °C requires a time period of heating of 195 hours or more and a temperature of 140 °C requires a time period of heating of 48 hours or more. Preferably, longer time periods than the minimum time periods are used.

The produced nanostructures may be coated, preferably with a coating comprising hydrophilic groups.

An advantage of this method is that a subsequent step of chromatographic purification of the produced nanostructures is usually not necessary.

The solution provided in step (a) may be provided by dissolving monomers having a purity of more than 80%, in a mixture of water and a lower alcohol. Preferably, the purity of the monomers is above 85%, or above 90%, or above 95%, or above 96%. Preferably, the monomer is 1,7-bis(triethoxysilyl)-4,4-bis(dimethoxyphosphonato)heptane, preferably purified as described herein.

The lower alcohol may be an alcohol comprising 1-8 carbon atoms. Preferably, the alcohol is ethylene glycol.

Preferably, $R^1$ and $R^2$ are lower alkyls, such as comprising 1-8 carbon atoms. More preferably, $R^1$ and $R^2$ are $-CH_3$.

Preferably, the $R^3$-groups are lower alkyls, such as comprising 1-8 carbon atoms. More preferably, the $R^3$-groups are $-CH_2-CH_3$.

Preferably, n=3.

In a specifically preferred embodiment, $R^1$ and $R^2$ are $-CH_3$, the $R^3$groups are $-CH_2-CH_3$, and n=3.

Thus, preferably, the monomer is 1,7-bis-(triethoxysilyl)-4,4-bis-(dimethylphosphonato)-heptane having a purity of more than 90%, optionally purified according to the method according to the present disclosure.

Thus, preferably, the monomer in step (a) is 1,7-bis-(triethoxysilyl)-4,4-bis-(dimethylphosphonato)-heptane having a purity of more than 90%, and wherein the monomer concentration is 30-40 mM, the solvent mixture is 10% water in ethylene glycol, and, in step (b), the temperature is 140 °C and the heating time is 45 to 50 hours.

Optionally, 1,7-bis-(triethoxysilyl)-4,4-bis-(dimethylphosphonato)-heptane has been purified according to the method according to the present disclosure,

The heating time may be 46 to 49 hours, such as 47 to 48 hours.

According to a seventh aspect, there is provided the use of a plurality of globular nanostructures according to the present disclosure and/or the product of a method according to the present disclosure as an intermediate in the production of a plurality of globular coated nanostructures.

According to an eighth aspect, there is provided the use of the plurality of globular nanostructures according to the present disclosure and/or the product of a method according to the present disclosure as an intermediate in the production of a pharmaceutical product. Preferably, the globular nanostructures comprise a coating. Preferably the coating comprises hydrophilic groups.

According to a ninth aspect, there is provided the use of a pharmaceutical composition comprising a plurality of globular nanostructures comprising a coating, preferably wherein the coating comprises hydrophilic groups, according to the present disclosure as a carrier of a radioactive isotope.

Other objectives, features and advantages of the present disclosure will appear from the following detailed description, from the experimental data, as well as from the attached claims. It is noted that the disclosure relates to all possible combination of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least one instance of said component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

## DEFINITION OF TERMS

The term "nanostructure" as used herein relates to an entity with a total size in the nanorange, i.e. up to 100 nm. As used herein the term excludes the structures often referred to as "core-shell nanoparticles" or just "nanoparticles" which usually have an inorganic core and an organic coating.

The term "globular" as used herein is meant to describe a shape such that the minor axis is no less than half of the major axis, i.e. the longest axis through the center (point of weight) of the structure is no more than twice the length of the shortest axis through the same point. For an explanatory illustration, not limiting this definition, see Fig. 1.

The term "globular nanostructure" as used herein relates to a nanostructure as discussed above having an essentially globular form or shape. This means that shapes such as flakes, rods, tubes, toroids, chains and ribbons are excluded.

The term "hydrodynamic diameter" as used herein refers to the diameter of the hypothetical hard sphere that diffuses at the same speed as the nanostructure in solution, i. e. the diameter of the equivalent hard sphere as calculated from the diffusion coefficient, according to the Stokes-Einstein equation. The term is also known as "Stokes diameter" or "Stokes-Einstein diameter". Hydration and shape are included in the behavior of the sphere. The diffusion coefficient is in turn calculated from e.g. the time dependent light scattering data obtained by the Dynamic Light Scattering (DLS) technique. Other technical methods to measure the diffusion coefficient of nanostructures are known to one skilled in the art and may be used instead. In those cases, measurements need to be referenced to the DLS-measurement. As

a comparison, bovine serum albumin is measured to have a hydrodynamic diameter of 6.9 nm by DLS in aqueous saline at pH 7 and room temperature. Depending on whether the number average, volume average, or scattered intensity average is used, the calculated values may be somewhat different. The volume average is generally the most useful since it shows which nanostructure size the bulk of the material has. The average diameters referred to in this text refers to volume averages as measured at 25 °C in aqueous saline or in 8% ethylene glycol (vol/vol) in saline.

The term "DLS" as used herein is an acronym for Dynamic Light Scattering, a particle sizing method, and may also be referred to as Photon Correlation Spectroscopy or Quasi-Elastic Light Scattering. The DLS sizes given as stated in the text and in the claims, if nothing else is specified, refers to the average of the volume weighted peak for a sample measured at 25 °C in aqueous solution with an ionic strength corresponding to 150 mM NaCl, also called saline, or in 8% ethylene glycol (vol/vol) in saline.

SEC-ELSD as used herein is an acronym for Size Exclusion Chromatography with Evaporative Light Scattering Detection.

The term "AFM" as used herein is an acronym for atomic force microscopy.

A "monomer" is a molecule that may bind covalently to other molecules of the same kind, (and optionally, other kinds) to form a polymer i.e. a macromolecule composed of several monomer residues.

The term "monomer residue" refers to the atoms derived from one monomer unit as incorporated into the larger polymer. Depending on how the monomers link up, all the atoms may be retained or some may be lost as the bonds form.

A "crosslink" refers to a link between two different chains in a polymer. It is usually formed by the reaction of multifunctional monomers (i.e. crosslinkers) added when forming the polymer. Crosslinks may also be introduced e.g. by radiation treatment, chemical means, or heat. For the materials of the current disclosure, which are network polymers of monomers with multiple branching capability the usual description of degree of crosslinking becomes somewhat unsuitable. For simplicity we just describe the average number of bonds between monomers. For example: if the monomer has the potential to form six bonds to other molecules and on average three of them are fulfilled, we avoid the term degree of crosslinking and state that we have "on average three bonds to other monomers" or sometimes "on average three bonds out of six possible to other monomers".

The term "crosslinked" refers to a structure formed after the formation of at least one crosslink.

A "branch point" is a position in a network polymer where a polymer chain branches to two or more branches.

The term "polymeric framework" as used herein relates to a covalently bound group of atoms forming a network structure with multiple crosslinks. Such polymeric frameworks are formed from the linking of suitable monomers and/or oligomers (i.e. a molecular complex consisting of a few monomer residues) via covalent bonds. Some examples of monomers are styrene, propylene, ethylene, tetrafluoroethylene, trifluoroethylene, difluoroethylene, methyl acrylate, ethyl acrylate, hydroxyethyl acrylate, acrylamide, methyl methacrylate, ethyl methacrylate, hydroxylethyl methacrylate, $H_2N-(CH_2)_p-COOH$, where p is 1 -10, 3- aminobenzoic acid, 4-aminobenzoic acid, N-vinyl pyrolidone and silicone precursors like $(CH_3COO)_2Si(CH_3)_2$. Some examples of polymer frameworks are formed from matching pairs of monomers like terephtalic acid + 1 ,4 diamino benzene, terephtalic acid + ethylene glycol, and $HCOO-(CH_2)_PCOOH$ + $H_2N-(CH_2)_q-NH_2$, where p and q independently are 1 -10. Oligomers with 2-10 monomer units linked can be used as precursors. Some examples of oligomers different from linked groups of the above monomers are cyclic or poly- cyclic silanes such as hexamethylcyclotrisiloxane, 2,4,6,8- tetramethylcyclotetrasiloxane, and decamethylcyclopentasiloxane. Some examples of crosslinkers are N,N'-methylenebis(acrylamide), O,O'-methylenebis(acrylic acid), epichlorohydrin, divinylbenzene, 1,3-divinyltetramethyldisiloxane, 1,3-phenylenediisocyanate, 3,3"-biphenyltetracarboxylic acid dianhydride, 1,4-butanedioldivinylether, tetraethoxysilane, oligosilicates such as metasilicate, or silsequioxanes, organosilanes such as bis(triethoxysilyl)methane, bis(triethoxysilyl)ethane, bis(triethoxysilyl)propane, bis(triethoxysilyl)butane, methyl triethoxysilane, ethyl triethoxysilane, and propyl triethoxysilane.

The polymeric framework constitutes the skeleton of a nanostructure according to the present disclosure. Such a nanostructure may be coated as described herein and in such cases, polymeric framework constitutes the central part of the coated nanostructure. The skilled person realizes that the random nature of the polymerization process causes the materials to be mixtures of many similar but in most cases not identical branching patterns, crosslink positions and molecular weights.

The term "chelating group" refers to a chemical group with the ability to successfully compete with water in electrostatic binding of a positively charged ion. A single chelating group does not bind very strongly but if several of them surround a positively charged ion, a synergistic strengthening of the binding occurs. This is called chelation.

The expression "arranged in a fashion that allows chelation" means that a number of chelating groups as defined above are arranged so that synergistic strengthening of the binding of a positively charged ion can occur. This can be obtained by either statistical means; when a large number of chelating groups are incorporated in a random polymer at such a density that at least a few of them find themselves in proximity so that they can bind the same positively charged ion; or by incorporating a preformed unit where the chelating groups are already sitting in close proximity. An example of the latter is the well-known chelator DOTA (dodecane tetraacetic acid).

The term "covalently attached", "covalently linked" and "covalently bound" as used herein are synonymous,

and the meaning thereof is well known to the skilled person.

The term "independently selected" as used herein in means that each of the different constituents mentioned before the term is selected from the group following after the term independently or separately from the selection of the other mentioned constituents.

The term "geminal bisphosphonate group" refers to two phosphonate groups separated by one carbon atom, i.e. the phosphonate groups are bound to the same carbon atom. Compounds comprising such a geminal bisphosphonate group are often referred to as 1,1 -bisphosphonates (or 1,1 - diphosphonates). The phosphonate groups in the geminal bisphosphonate group may be substituted.

The term "radionuclide" refers to an unstable form of a chemical element that decays radioactively, resulting in the emission of $\alpha$, $\beta$ and/or y radiation.

As used herein, the expression "radionuclides for imaging and/or radiotherapy" refers to actinium-225 ($^{225}$Ac); copper-62 ($^{62}$Cu); copper-64 ($^{64}$Cu); copper-67 ($^{67}$Cu); gallium-67 ($^{67}$Ga); gallium-68 ($^{68}$Ga); holmium-166 ($^{166}$Ho); indium-111 ($^{111}$In); lead-212 ($^{212}$Pb); lutetium-177 ($^{177}$Lu); radium-223 ($^{223}$Ra); rhenium-186 ($^{186}$Re); rhenium-188 ($^{188}$Re); rubidium-82 ($^{82}$Rb); samarium-153 ($^{153}$Sm); strontium-89 ($^{89}$Sr); technetium-99m ($^{99m}Tc^{3+}$); thallium-201 ($^{201}$Tl); thorium-227 ($^{227}$Th); yttrium-86 ($^{86}$Y); yttrium-90 ($^{99}$Y); and zirconium-89 ($^{89}$Zr). The expression "a radionuclide for imaging and/or radiotherapy" also encompasses combinations of two or more of the above mentioned radionuclides.

As used herein, the expression "radionuclides for imaging" refers to copper-62 ($^{62}$Cu); copper-67 ($^{67}$Cu); gallium-67 ($^{67}$Ga); gallium-68 ($^{68}$Ga); indium-111 ($^{111}$In); lutetium-177 ($^{177}$Lu); rhenium-186 ($^{186}$Re); rubidium-82 ($^{82}$Rb): technetium-99m ($^{99m}Tc^{3+}$); Thallium-201 ($^{201}$Tl); yttrium-86 ($^{86}$Y) and zirconium-89 ($^{89}$Zr). The expression "a radionuclide for imaging" also encompasses combinations of two or more of the above mentioned radionuclides.

As used herein, the expression "radionuclides for PET imaging" refers to copper-62 ($^{62}$Cu); gallium-68 ($^{68}$Ga); rubidium-82 ($^{82}$Rb); yttrium-86 ($^{86}$Y), and zirconium-89 ($^{89}$Zr). The expression "a radionuclide for PET imaging" also encompasses combinations of two or more of the above mentioned radionuclides.

As used herein, the expression "radionuclides for SPECT imaging" refers to gallium-67 ($^{67}$Ga); indium-111 ($^{111}$In); technetium-99m ($^{99m}Tc^{3+}$); lutetium-177 ($^{177}$Lu), and thallium-201 ($^{201}$Tl). The expression "a radionuclide for SPECT imaging" also encompasses combinations of two or more of the above mentioned radionuclides.

As used herein, the expression "radionuclides for radiotherapy" refers to actinium-225 ($^{225}$Ac); copper-64 ($^{64}$Cu); copper-67 ($^{67}$Cu); holmium-166 ($^{166}$Ho); lead-212 ($^{212}$Pb); lutetium-177 ($^{177}$Lu); radium-223 ($^{223}$Ra); rhenium-186 ($^{186}$Re); rhenium-188 ($^{188}$Re); samarium-153 ($^{153}$Sm); strontium-89 ($^{89}$Sr); thorium-227 ($^{227}$Th) and yttrium-90 ($^{90}$Y). The expression "a radionuclide for radiotherapy" also encompasses combinations of two or more of the above mentioned radionuclides.

As used herein, the expression "radionuclides for PET imaging and radiotherapy" refers to actinium-225 ($^{225}$Ac); copper-62 ($^{62}$Cu); copper-64 ($^{64}$Cu); copper-67 ($^{67}$Cu); gallium-68 ($^{68}$Ga); holmium-166 ($^{166}$Ho); lead-212 ($^{212}$Pb); lutetium-177 ($^{177}$Lu); radium-223 ($^{223}$Ra); rhenium-186 ($^{186}$Re); rhenium-188 ($^{188}$Re); rubidium-82 ($^{82}$Rb); samarium-153 ($^{153}$Sm); strontium-89 ($^{89}$Sr); thorium-227 ($^{227}$Th); yttrium-90 ($^{90}$Y) and zirconium-89 ($^{89}$Zr). The expression "a radionuclide for PET imaging and radiotherapy" also encompasses combinations of two or more of the above mentioned radionuclides.

As used herein, the expression "radionuclides for SPECT imaging and radiotherapy" refers to actinium-225 ($^{225}$Ac); copper-64 ($^{64}$Cu); copper-67 ($^{67}$Cu); gallium-67 ($^{67}$Ga); holmium-166 ($^{166}$Ho); indium-111 ($^{111}$In); lead-212 ($^{212}$Pb); lutetium-177 ($^{177}$Lu); radium-223 ($^{223}$Ra); rhenium-186 ($^{186}$Re); rhenium-188 ($^{188}$Re); samarium-153 ($^{153}$Sm); strontium-89 ($^{89}$Sr); technetium-99m ($^{99m}Tc^{3+}$); thallium-201 ($^{201}$Tl); thorium-227 ($^{227}$Th) and yttrium-90 ($^{90}$Y). The expression "a radionuclide for SPECT imaging and radiotherapy" also encompasses combinations of two or more of the above mentioned radionuclides.

The term "bioinert" as used herein refers to a material that is biocompatible, i.e. harmless to mammals and mammalian cells and at the same time stable to degradation *in vivo,* in a human (less than 10% degraded) for periods of one week or more.

The term "oxysilane" as used herein refers to any compounds with one or more oxygen atoms attached to the silicon atom. Non-limiting examples thereof are:

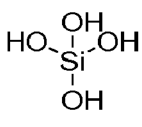 , 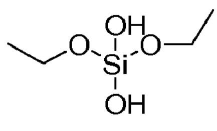 and 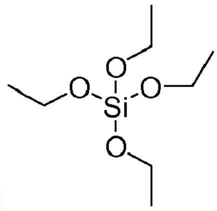 .

The term "organosilane" as used herein refers to organic compounds containing one or more carbon-silicon bond(s).

The terms "hydrocarbon" and "hydrocarbon chain" are used herein to denote an organic residue consisting of hydrogen and carbon. The hydrocarbon may be fully saturated or it may comprise one or more unsaturations. The hydrocarbon in accordance with the present disclosure may contain any number of carbon atoms between 1 and 50.

The term "alkyl" as used herein refers to a straight or branched hydrocarbon chain fully saturated (no double or triple bonds) hydrocarbon group. The alkyl group may in the present text have 1-15 carbon atoms. Typical alkyl groups include, but are in no way limited to, methyl, ethyl, propyl, isopropyl, butyl, isobutyl, tertiary butyl, pentyl, hexyl and the like.

The term "lower alkyl" as used herein refers to an alkyl having 1-8 carbon atoms.

The term "lower alcohol" as used herein refers to an alcohol having 1-8 carbon atoms.

The term "low boiling alcohol" as used herein refers to an alcohol having a boiling point below 160°C.

The term "lower alkane" as used herein refers to a straight or branched, fully saturated (no double or triple bonds), hydrocarbon having 1-8 carbon atoms. When the term is used in regards to solvents, it refers to a straight or branched, fully saturated (no double or triple bonds), hydrocarbon having 4-8 carbon atoms.

Numerical ranges: Whenever it is used herein, unless otherwise stated, a numerical range such as "1 to 8" or "1-8" refer to each integer in the given range; e.g., "1 to 8 carbon atoms" and "1-8 carbon atoms" mean that the alkyl group may consist of 1 carbon atom, 2 carbon atoms, 3 carbon atoms, etc., up to and including 8 carbon atoms. There are, however some exceptions which are clear to the skilled persons. In particular, whenever a range is given herein for a molar ratio, such as the Si/P molar ratio in the nanostructures, for a diameter or size, for a pH, for a period of time, for a concentration, for an osmolality or for a temperature, the range includes also all decimal numbers falling within the range, including the upper and lower limits.

As used herein, the term "alkoxy" refers to the formula -OR wherein R is a lower alkyl, e.g. methoxy, ethoxy, n-propoxy, 1-methyl ethoxy (isopropoxy), n-butoxy, iso-butoxy, sec-butoxy, tert-butoxy, amyloxy, iso-amyloxy and the like. An alkoxy group in accordance with the present disclosure may be optionally substituted.

As used herein the term "aryl" refers to a carbocyclic (i.e. all carbon) ring or two or more fused rings (i.e. rings that share two adjacent carbon atoms) that have a fully delocalized pi-electron system. Examples of aryl groups include, but are not limited to, benzene, naphthalene and azulene. An aryl group in accordance with the present disclosure may be optionally substituted, e.g., phenoxy, naphtha- lenyloxy, azulenyloxy, anthracenyloxy, naphthalenylthio, phenylthio and the like. An aryloxy may be optionally substituted.

The term "conjugate" as used herein refers to a molecular entity that is a fluorescence marker, dye, spin-label, radioactive marker, peptide, ligand to a biological receptor, chelate, enzyme inhibitor, enzyme substrate, antibody or antibody related structure.

The term "coated nanostructure" (or nanostructure having a coating) is used to describe a material that can be produced from the nanostructures of the current disclosure by the addition of one or more layer(s) of additional materials. Often such a coated nanostructure is intended to be used for radioisotope therapy.

The term "dispersity" is used where previously the term polydispersity was used. For most applications it is important to have as narrow a distribution of sizes as possible around the average i.e. a low dispersity. For a sample with completely uniform size the dispersity is 1 and for non-uniform samples it will be larger than 1. The dispersity can be calculated in different ways, either as the ratio of Mw/Mn or similarly $M_v/M_n$, where Mw is the weight-average molecular weight, $M_n$ is number-average molecular weight, $M_v$ is the volume-average molecular weight. Herein we analogously define dispersity, $Đ_d$, for a sample of nanostructures as $Đ_d = d_v/d_n$ were $d_v$ is the volume-average diameter as measured by DLS and $d_n$ is the number average diameter as measured by DLS. Another way of describing the dispersity of a material is to compare to known size references. As a non-limiting example given here as an illustration, suitable size references can be bovine serum albumin (BSA), or Thyroglobulin or empty Cowpea Mosaic Virus (CPMV) VLPs.

The term "growth rate" or "rate of growth" as used herein refers to the first derivative of the size of a nanostructure with respect to time. For convenience the rate of growth is often approximated as the change in size of a nanostructure between two measurements, such as the change in average hydrodynamic diameter, $\Delta d$, divided by the time elapsed between the two measurements, $\Delta t$. The size of the nanostructure may be determined by any of a number of methods known to one skilled in the art, such as DLS or SEC. The unit of measurement of the growth rate will depend on the method used to determine the size but may take such units as nm/h if DLS is used for size determination or minutes/hour if SEC is used for size determination.

As used herein, the term "vol/vol" or "v/v" denotes volume/volume.

## BRIEF DESCRIPTION OF THE DRAWINGS

By way of example, embodiments of the present teaching will now be described with reference to the accompanying drawings, in which:

Fig. 1 is an explanatory illustration of a globular shape.

Fig. 2 is an illustration of how chelating groups (illustrated as "U") in a network polymer (represented by the thick black curved line) may distribute to form ion binding sites of various binding strength.

Fig. 3 shows $^{1}H$ NMR spectrum of long boiled (top trace) and short boiled (bottom trace) nanostructures.

Fig. 4 shows $^{31}P$ NMR of long boiled and short boiled nanostructures (bottom to top: short boiled with 1H-decoupling, short boiled without 1H-decoupling, long boiled with 1H-decoupling, long boiled without 1H-decoupling) showing how the phosphonate esters present in the short boiled nanostructures (two bottom traces) hydrolyzes to form only one phosphonate peak in the long boiled nanostructure (two top traces).

Fig. 5 shows HPLC chromatogram of A) crude 7-bis-(triethoxysilyl)-4,4-bis-(dimethylphosphonato)-heptane of a purity of 54 %, and B) purified 7-bis-(triethoxysilyl)-4,4-bis-(dimethylphosphonato)-heptane of a purity of 97 %, C) Details from A and B overlaid to show relative amounts, the crude material as the thin line and the purified material in the thicker line.

Fig. 6 shows SEC chromatogram of particles made according to Example 1B. The particles have a mean size of 18.2 nm. The retention time (tR) is in minutes. The three peaks in thin light gray color are CPMV, $t_R$~8.28 min, Thyro, $t_R$~9.04 min and BSA, $t_R$~10.05 min, respectively.

Fig. 7 is an AFM image of particles made according to Example 1B. The individual particles fulfill the globular shape definition. Some aggregates of particles are also visible.

Fig. 8 is a scheme of the production process for producing nanostructures according to the present disclosure.

## DETAILED DESCRIPTION

The present disclosure relates to globular nanostructures, methods of purifying monomers used in the production of such nanostructures, as well as methods for producing such nanostructures and specific uses of such nanostructures.

Specific aspects and embodiments of the present disclosure will be described in detail below.

### *Globular nanostructures*

A plurality of globular nanostructures according to the present disclosure can be characterized by average hydrodynamic diameter and dispersity. Other informative measures may involve average molecular weight, and density. The molecular weight for a given globular nanostructure can be calculated as the product of the nanostructure volume, calculated according to a geometric formula known in the art, and the density.

The range of sizes of the nanostructures according to the present disclosure is, limited from below by their ability to chelate the radioisotope with high affinity and keep it bound for many days after administration to a living organism. Example 4 below shows that there is a correlation between the size of the nanostructures according to the present disclosure and their chelating strength. The chelating strength is considered adequate for sizes above 13 nm in diameter, particularly for sizes above 15 nm.

The upper size limit is set by the ability of the coated nanostructures, derived from the globular nanostructures according to the present disclosure, to penetrate from the blood stream into tumor tissue in the body of an organism. It has been found that it is more advantageous to use globular nanostructures at the lower end of the feasible size range, such as between 13 and 25 nm, such as between 16 and 20 nm, as the precursors of coated nanostructures since the diffusion resistance in tissue is high. The diffusion resistance depends on the size of the nanostructure and for entities above 100 nm diameter, it has been found that the diffusion resistance is so high that the dose delivered locally to a tumor is too small for being useful for many clinical purposes.

When used in imaging or therapy, the nanostructures preferably comprise a coating as described herein. The biodistribution of coated nanostructures according to the present disclosure has been investigated (Example 6. The data shown in Example 6 indicates that there is a sizeoptimum of the coated nanostructures around 30 nm. It is estimated that size of the coated nanoparticles should be no more than 100 nm, preferably less than 90 nm, more preferred less than 60 nm, even more preferred less than 35 nm. The coating adds at least 5 to 10 nm to the diameter so the upper limit for the core nanostructures (uncoated nanostructures) of the present disclosure is 90 nm. Thus, the nanostructures according to the present disclosure are less than 90 nm, such as less than 70 nm, such as less than 50 nm, such as less than 30 nm such as less than 25 nm, such as less than 22 nm, such as less than 20 nm.

In one embodiment, a plurality of nanostructures of the current disclosure with an average size of 18 nm has 95% of the population between protein BSA and virus-like-particle CPMV when measured by SEC-ELSD.

Example 7 and Fig.7 describe a plurality of nanostructures of the current disclosure with an average size of 18.2 nm according to DLS having 1.6% of the peak area in a SEC-ELSD chromatogram with size above reference CPMV and 2.8% below reference BSA.

Example 1 describes how to select conditions to produce the nanostructures of the current disclosure within the given size range.

The nanostructures of the current disclosure comprise a polymeric framework of monomer residues, wherein at least 90% of the monomer residues comprise two geminal chelating groups, each chelating group independently being a group according to Formula (I), $-PO(OR^1)(OR^2)$, wherein $R^1$ and $R^2$ are independently selected from the group consisting of a negative charge and H; and - denotes an internal bond in the monomer residue.

The polymeric framework may be a homopolymer of a single monomer or a copolymer of two or more different monomers.

The polymeric framework may in principle be constructed from a large number of well-known monomers as can be found in any book on polymer chemistry.

Specifically, in preferred embodiments, the nanostructures of the present disclosure comprise polymers with a randomly branching and cross-linking pattern, as opposed to cascade polymers such as dendrimers or arborols, or macromolecules such as proteins, which all have molecularly well-defined structures where essentially all molecular entities are identical. The advantage of this approach is that it is possible to produce nanostructures with a minimum size as low as 13 nm in a cost-effective, reliable and relatively easy way. Although it is possible to reach the desired minimum size for a nanostructure with well-defined molecular entities, it is very costly and cumbersome to do so. An example would e.g. be the largest dendrimer that seems to be commercially available, PAMAM-G10, which is stated to have a hydrodynamic diameter of 13.5 nm, at a cost of about € 4,000 for a 100 mg research sample (Sigma-Aldrich, prod no. 536776) reaching only the lower part of the desired size range of 13-90 nm of the nanostructures according to the present disclosure. The production cost of nanostructures of the current disclosure is foreseen to be less than 1% of the above price. Furthermore, a particular advantage of the approach of the current disclosure is that chromatographic purification is usually not necessary.

In a specific embodiment, wherein the nanostructures comprise polymers with a randomly branching and cross-linking pattern, the number of bonds between the monomer residues is unusually high for such polymers. In such cases, the number of bonds between the monomer residues are on average more than two bonds per monomer; or more than three bonds per monomer. Even such high numbers as 4 to 5 or, less than, but close to 6, may be contemplated. It is obvious to the person skilled in the art that even if monomers with potential for crosslinking or branching are used as monomers to produce a nanostructure according to the present disclosure, not all of the potential will be fulfilled in practice so some residual groups with potential for crosslinking or branching will be left in the structure of said central part.

It is generally difficult to precisely determine the average number of bonds between monomer residues in nanostructures according to the present disclosure, but information from elemental composition, density measurements, NMR and AFM puts some constraints on it and it is clear the number of bonds between monomer residues is very high as described above.

In one embodiment, the nanostructures comprise a homopolymer where there are six groups with potential for bonding in the monomer and between three and five of the groups actually form bonds to other monomer residues.

In another embodiment, the average number of bonds between the monomer residues is between three and 5.9.

Preferably, at least 90% of the monomer residues are residues according to Formula (II):

$$\{(OR^1)(OR^2)PO\}_2\text{-}(C)\{(CH_2)_nSi(OR^3)_3\}\{(CH_2)_nSi(OR^3)_3\} \qquad \text{(II)}$$

wherein each $R^1$ and $R^2$ is independently selected from the group consisting of a negative charge and H; each $R^3$ is independently selected from the group consisting of a negative charge, H and a covalent bond to the polymeric framework; wherein at least 3 $R^3$ are bonds to the polymeric framework; and n is an integer between 1 and 5.

Preferably, at least 3 of the $R^3$-groups are bonds to the polymeric framework.

Preferably n=3.

Preferably, 3 $R^3$-groups are bonds to the polymeric framework; and n = 3.

Preferably, 4 $R^3$-groups are bonds to the polymeric framework; and n = 3.

Preferably, 5 $R^3$-groups are bonds to the polymeric framework; and n = 3.

Preferably, all 6 $R^3$-groups are bonds to the polymeric framework; and n = 3.

Preferably, all $R^3$-groups are independently selected from the group consisting of a negative charge, H, or a bond to the polymeric framework.

Preferably, all $R^3$-groups are independently selected from the group consisting of a negative charge, H, or a bond to the polymeric framework; and n = 3.

Typically, there are at least a two hundred chelating groups in each nanostructure, arranged in such a fashion that allows the chelation of one or more multiply charged cations.

The chelating groups may be randomly distributed throughout the nanostructure and rely on chance to arrange themselves in a way that allows chelation of the multiply charged cations (see Fig. 2). When relying on chance it is preferable to incorporate a large excess of chelating groups in the nanostructure to get an increased probability of forming a cluster of chelating groups with high chelating ability.

As stated above, the chelating groups present in nanostructures of the present disclosure are groups according

to Formula (I) as defined above. When incorporated into a polymeric framework, especially when incorporated as geminal phosphonates, and allowed to bind multiply charged cations, these chelating groups bind cations strongly. In example 4 is shown how the nanostructures comprising geminal bisphosphonates compete favorably to the strong chelator EDTA.

Preferably, the phosphonate groups are essentially completely hydrolyzed to their acid form and subsequently ionized to some extent from partial to complete according to the pH value of the surrounding medium.

If the phosphonate groups are partially present in their ester form, it has been found that the nanostructures bind metal ions less strongly.

Nanostructures comprising such phosphonate groups bind said multivalent cations best a neutral or basic pH. This indicates that it is, at least in part or sometimes or even completely, the anionic form of the hydrolyzed phosphonate that plays an important part in the binding of the metal ions.

It is advantageous if the phosphonates are predominantly or completely hydrolyzed to phosphonic acids when incorporated in the nanostructure. In example 8 and Fig. 4 is shown NMR data supporting that the phosphonates are essentially completely hydrolyzed.

In one embodiment, the phosphonates are hydrolyzed to phosphonic acids to a large degree, such as more than 50%, or more than 90% or more than 95%, when present in the nanostructure.

Not only phosphonate esters or acids but also phosphonic amides may be contemplated as part of the material or to be used as starting material.

In another embodiment, a dried sample of said nanostructure has a density of 1.3 -1.7 g/cm$^3$, such as 1.4-1.68 g/cm$^3$, such as 1.5-1.67, such as 1.6-1.65 g/cm$^3$.

The very high density (1.469 g/cm$^3$) of a dried sample of the nanostructures from example 1 are in line with the typically very high densities of phosphonic acids (1.3-2.0 g/cm$^3$) as opposed to the more normal densities of phosphonic esters (typically around 1.1 g/cm$^3$).

***Coated globular nanostructures***

In some embodiments the nanostructures comprise a coating. The coating contributes to the biocompatibility of the nanostructures. Preferably, the coating is a hydrophilic coating. Typically, a coated nanostructure as described herein has an average hydrodynamic diameter of 18-100 nm, such as 20-50 nm, such as 25-35 nm.

The coating may comprise polyethylene glycol (PEG). Each polyethylene glycol chain may comprise between 10 and 150 ethylene glycol residues, preferably between 20 and 100, such as between 30 and 50 ethylene glycol residues, such as 45 ethylene glycol residues.

Preferably, the coating comprises coating monomer residues comprising 2 silicon atoms and 2 polyethylene glycol chains, each polyethylene glycol chain comprising between 20 and 100 ethylene glycol units.

In a specific embodiment, the coating comprises coating monomer residues comprising one or multiple polyethylene glycol chains and one or multiple silicon atoms, joined through an organic linker. The organic linker may be a hydrocarbon. The organic linker may also comprise ether bonds. The silicon atoms in the monomer may be present in reactive siloxane groups, such as $-Si(OEt)_3$, $-Si(OMe)_3$, or $-SiCl_3$.

When the coating comprises coating monomer residues comprising silicon atoms, the coating monomer residues are bound to the rest of the nanostructure through siloxane bonds.

The coating may be grafted onto the nanostructures through a reaction between the nanostructure and coating monomers comprising reactive siloxane groups, such as $-Si(OEt)_3$, $-Si(OMe)_3$, or $-SiCl_3$.

Preferred coating monomers are $(m\text{-}PEG_xOCH_2)_2C]\text{-}CH_2CH_2CH_2\text{-}Si(OEt)_3]$, wherein m is short for methyl and x is an integer between 10 and 150, such as between 20 and 100, such as between 30 and 50, such as 45 ethylene glycol residues.

Other preferred coating monomers are $m\text{-}PEG_x\text{-}CH_2CH_2CH_2\text{-}Si(OEt)_3$, wherein m is short for methyl and x is an integer between 10 and 150, such as between 20 and 100, such as between 30 and 50, such as 45 ethylene glycol residues.

Other preferred coating monomers are 1-(ω-methyl-(ethyleneoxy)$_x$-methyl)-3,5-bis(3-(triethoxysilyl)propyloxy)-benzene, wherein x is an integer between 10 and 150, such as between 20 and 100, such as between 30 and 50, such as 45; and 1,7-bis(triethoxysilyl)-4,4-bis(ω-methyl-(ethyleneoxy)$_x$-methyl)-heptane wherein x is an integer between 10 and 150, such as between 20 and 100, such as between 30 and 50, such as 45.

***Pharmaceutical composition comprising coated globular nanostructures***

A pharmaceutical composition comprising a plurality of globular nanostructures, further comprising a coating, according to the present disclosure is preferably prepared as a solution.

Typically, when used for imaging and/or treatment of cancer, the globular nanostructures further comprise radioactive isotope. Depending on the application, different radioactive isotopes are used. The composition may comprise

nanostructures comprising one single radioactive isotope or two or more different types of radioactive isotopes. Radioactive isotopes, also referred to as radionuclides, suitable for different applications have been listed above.

In one embodiment, said alkoxysilanes are separated by 1-10 carbon atoms or 3-9 carbon atoms.

In another embodiment, said alkoxysilanes are separated by 7 carbon atoms.

In a preferred embodiment, the polymeric framework is derived from 1,7-bis-(triethoxysilyl)-4,4-bis-(dimethylphosphonato)-heptane. The polymeric framework may have been formed by a hydrolytic condensation polymerization.

In another preferred embodiment, the polymeric framework is derived from 1,7-bis-(triethoxysilyl)-4,4-bis-(dimethylphosphonato)-heptane with a purity above 80%, or above 85%, or above 90%, or above 95%, or above 96%.

When the nanostructures have been formed by the linking of a multitude of monomers into a polymeric network, the residues of the monomers are designated monomer residues. They still retain the underlying covalent bond pattern of the original free monomers but the formation of the linkages modify the groups directly involved in said linkages.

***Method for purifying 1,7-bis(triethoxysilyl)-4,4-bis(dimethoxy-phosphonato)heptane***

As discussed below, the purity of the monomer affects the quality of the nanostructure produced by the method disclosed below. (Example 1).

As discussed below, when nanostructures are produced by the method disclosed below, the use of monomers of a higher purity results in higher quality nanostructures.

Thus, a method for purifying 1,7-bis-(triethoxysilyl)-4,4-bis-(dimethylphosphonato)-heptane to very high purity, such as above 80%, or above 85%, or above 90%, or above 95%, or above 96% has been developed.

The method is suitable for a moisture sensitive, reactive, high boiling, thermally sensitive oil on a large scale, such as multi-kilo production in an industrial setting. Notably, all standard purification methods are unsuitable for such materials. Chromatography on silica fails because of the reactive nature of the triethoxy silanes and large-scale reversed phase chromatography fails because of the need for an aqueous mobile phase. The same holds for extraction. Crystallization doesn't work for oils and the material is too thermally sensitive to stand the prolonged heating conditions of vacuum distillation (shown in Example 3).

The method for purifying 1,7-bis(triethoxysilyl)-4,4-bis(dimethoxy-phosphonato)heptane of the present disclosure comprises the steps of:

(a) providing a solution of impure 1,7-bis(triethoxysilyl)-4,4-bis(dimethoxyphosphonato)heptane in a polar aprotic solvent;
(b) separating the solution of step (a) from insoluble matter;
(c) concentrating the solution obtained in step (b), thereby providing a residue;
(d) dissolving the residue obtained in step (c) in a non-polar solvent;
(e) separating the solution obtained in step (d) from insoluble matter;
(f) removing water from the solution obtained in step (e);
(g) concentrating the dried solution obtained in step (f), resulting in a second residue;
(h) subjecting the residue obtained in step (g) to a short path, pass-through vacuum distillation; and
(i) collecting the pass-through fraction from step (h), comprising purified 1,7-bis(triethoxysilyl)-4,4-bis(dimethoxyphosphonato)heptane.

The aprotic solvent in step (a) may be a nitrile, e.g. acetonitrile and propionitrile; a ketone, e.g. acetone and methylethylketone; an ester, e.g. ethyl actetate and isopropyl acetate; or a polar ether, e.g. THF (terahydrofuran) and Me-THF (methyl-tetrahydrofuran). In a preferred embodiment, the aprotic solvent in step (a) is acetonitrile.

Specifically, step (a) may be performed by dissolving a crude quality of 1,7-bis-(triethoxysilyl)-4,4-bis-(dimethylphosphonato)-heptane in acetonitrile. The impure 1,7-bis(triethoxysilyl)-4,4-bis(dimethoxy-phosphonato)heptane may be dissolved in acetonitrile at a concentration between 5% (w/v) and 20% (w/v), such as between 7% (w/v) and 15% (w/v), such as 10% (w/v), (w/v) denoting weight/volume.

The separation step (b) may be performed by e.g., filtration or sedimentation and decantation.

Step (c) may be performed by evaporation.

The non-polar solvent in step (d) may be a cyclic or non-cyclic hydrocarbon or a mixture of hydrocarbons.

In step (d) the residue obtained in step (c) may be dissolved in an alkane.

In step (d) the residue obtained in step (c) may be dissolved in a lower alkane.

Examples of suitable cyclic hydrocarbons are cyclohexane and cycloheptane.

Examples of suitable non-cyclic hydrocarbons are pentane, hexane and heptane. Specifically, in step (d) he residue obtained in step (c) may be dissolved in heptane. The residue obtained in step (c) may be dissolved in heptane at a concentration such as between 5% (w/v) and 20% (w/v), such as between 7% (w/v) and 15% (w/v), such as 10% (w/v), (w/v) denoting weight/volume).

The separation step (e) may be performed by e.g. filtration or sedimentation and decantation.

In step (f), the water may be removed by evaporation or by drying the solution obtained in step (e) over a drying agent such as 4 Å molecular sieves. When molecular sieves are used, step (g) is performed by first separating the solution from the molecular sieves.

The short path, pass-through vacuum distillation in step (h) is preferably performed by falling film vacuum removal of impurities at a temperature ranging from 150 °C to 190 °C, such as 160 °C to 180 °C, or 165 °C to 175 °C.

The precipitation steps (steps (a) and (d)) are advantageous to remove oligomeric materials which may otherwise clog up the distillation setup. Notably, normal vacuum distillation is not feasible for 1,7-bis-(triethoxysilyl)-4,4-bis-(dimethylphosphonato)-heptane since it was found that this compound is rapidly broken down at the required temperature for boiling, even under vacuum (Example 3).

To solve this difficult purification problem, the two-stage process involving selective precipitation of impurities followed by falling film distillation was developed. The specialized method of falling film distillation exposes the material to high temperatures for only a few seconds which is too short for thermal degradation. Some other versions of the wider concept "short path distillation" with short residence times at high temperature are also suitable, including wiped film distillation methods.

To remove the lower boiling impurities, in step (h), the intermediate purity mixture is passed through the distillation apparatus allowing the impurities to evaporate.

The falling film distillation has the advantage that it is a technique that is available from lab scale to production plant scale.

The distillation may be carried out in two alternative ways, including one step or including two steps: In a first step, the temperature of the heating element is set to a temperature such as from 150 °C to 165 °C and the crude product is passed through the equipment. Then the lower boiling impurities of the character of lacking some structural feature, such as a silyl group, are removed and collected as distillate and the purified product is retained. In an optional, second step the heating element is set to a higher temperature such as from 170 °C to 190 °C and the once purified product is passed through the equipment. Then the product is vaporized and collected as distillate and non-volatiles are retained. This second step is advantageous since the production of many silanes, such as 1,7-bis-(triethoxysilyl)-4,4-bis-(dimethylphosphonato)-heptane, may involve a platinum catalyzed hydrosilylation step and may contain unacceptable amounts of platinum residues. Those are non-volatiles and are removed by the second step.

In a specific embodiment, 1,7-bis-(triethoxysilyl)-4,4-bis-(dimethyl-phosphonato)-heptane is purified by a sequence of dissolving the crude material in acetonitrile in step (a) and separating the insoluble material by filtration (step (b)) followed by evaporation of the solvent (step(c)), followed by dissolving the resulting material in a lower alkane in step (d), and separating the insoluble material by filtration (step (e)), followed by removing water (step (f)) by molecular sieves and removing the molecular sieves by filtration, followed by evaporation of the solvent (step (g)), followed by falling film distillation under vacuum in step (h) with the heating element heated to between 150 °C and 175 °C and collecting the concentrate.

In another specific embodiment, 1,7-bis-(triethoxysilyl)-4,4-bis-(dimethylphosphonato)-heptane is purified by a sequence of dissolving the crude material in acetonitrile at a concentration between 7% (w/v) and 15% (w/v) in step (a) and separating the insoluble material by sedimentation and decantation followed by filtration (step (b)) followed by evaporation of the solvent (step (c)), followed by dissolving the resulting material in heptane at a concentration between 7% (w/v) and 15% (w/v) in step (d) and separating the insoluble material by sedimentation and decantation (step (e)) followed by drying over activated molecular sieves followed by removing the molecular sieves by filtration (step (f)) followed by evaporation of the solvent (g), followed by falling film distillation under vacuum in step (h) with the heating element heated to 165 °C and collecting the concentrate (i).

In another specific embodiment, 1,7-bis-(triethoxysilyl)-4,4-bis-(dimethylphosphonato)-heptane is purified by a sequence of dissolving the crude material in acetonitrile at a concentration of 10% (w/v) in step (a) and separating the insoluble material by sedimentation and decantation followed by filtration (step (b)) followed by evaporation of the solvent (step (c)), followed by dissolving the resulting material in heptane at a concentration of 10% (w/v) in step (d) and separating the insoluble material by sedimentation and decantation followed by filtration (step (e)) followed by drying over activated molecular sieves followed by removing the molecular sieves by filtration (step (f)) followed by evaporation of the solvent (g), followed by falling film distillation under vacuum in step (h) with the heating element heated to 165 °C and collecting the concentrate (i).

Alternatively, 1,7-bis-(triethoxysilyl)-4,4-bis-(dimethylphosphonato)-heptane may be purified by a sequence of (i) dissolving the crude material in heptane at a concentration of 10% (w/v) as in step (d) above and (ii) separating the insoluble material by sedimentation and decantation followed by filtration (as in step (e) above) followed by (iii) evaporation of the solvent (as in step (g) above), followed by (iv) dissolving the resulting material in acetonitrile at a concentration of 10% (w/v) as in step (a) above and (v) separating the insoluble material by sedimentation and decantation followed by filtration (as in step (b) above) followed by (vi) drying over activated molecular sieves followed by removing the molecular sieves by filtration (as in step (f) above) followed by (vii) evaporation of the solvent (as in step (c) above),

followed by (viii) falling film distillation under vacuum in step (as in step (h) above) with the heating element heated to 165 °C and (ix) collecting the concentrate (as in step (i) above).

It is conceivable to employ only the short path, pass-through vacuum distillation in used in step (h) to purify impure 1,7-bis(triethoxysilyl)-4,4-bis(dimethoxyphosphonato)heptane, especially if the impure 1,7-bis(triethoxysilyl)-4,4-bis(dimethoxyphosphonato)heptane is comprises only small amounts of macromolecular impurities. However, it has been found that when known methods for the synthesis of 1,7-bis(triethoxysilyl)-4,4-bis(dimethoxyphosphonato)heptane are used, there is always oligomeric impurities present in the impure 1,7-bis(triethoxysilyl)-4,4-bis(dimethoxy-phosphonato)heptane.

As explained above, step (h) may include a further step of purifying 1,7-bis-(triethoxysilyl)-4,4-bis-(dimethylphosphonato)-heptane by falling film distillation under vacuum with the heating element heated above 185 °C and collecting the distillate. In such cases, the resulting purified material 1,7-bis-(triethoxysilyl)-4,4-bis-(dimethylphosphonato)-heptane has a lower concentration of inorganic impurities.

One type of impurities present in impure 1,7-bis-(triethoxysilyl)-4,4-bis-(dimethylphosphonato)-heptane are the side products that form during the production of the monomer. HPLC analysis of a crude preparation of 1,7-bis-(triethoxysilyl)-4,4-bis-(dimethylphosphonato)-heptane indicates a purity of 54% and in Fig. 5A the HPLC trace of such a sample is shown. In Fig. 5B a sample of material that has been purified to 97% according to the method described above (for details see example 2) is shown. It is evident that the purity has been improved. This is an important advantage when 1,7-bis-(triethoxysilyl)-4,4-bis-(dimethylphosphonato)-heptane later is used in the production of nanostructures according to the present disclosure.

1,7-bis(triethoxysilyl)-4,4-bis(dimethoxyphosphonato)heptane purified as described herein may be used for producing a plurality of globular nanostructures according to the present disclosure.

*Method for producing a plurality of globular nanostructures*

A method for producing a plurality of globular nanostructures according to the present disclosure is outlined in Figure 8. It has been found that this method results, even when performed on a large scale, in nanostructures of a quality suitable for use in imaging and radiotherapy and having specific size and dispersity.

In short, the method for producing a plurality of globular nanostructures according to the present disclosure involves the dissolution (step 001) of the monomer in a water/solvent mixture (002) and heating (003) the solution for an extended period of time (Figure 8).

In detail, the method comprising the steps of:

(a) providing a solution comprising monomers in a mixture of water and a lower alcohol, wherein the monomers are monomers according to Formula (II)

$$\{(OR^1)(OR^2)PO\}_2\text{-}(C)\{(CH_2)_nSi(OR^3)_3\}\{(CH_2)_nSi(OR^3)_3\} \qquad (II)$$

wherein

each $R^1$ and $R^2$ is independently selected from the group consisting of lower alkyls and aryl; and
each $R^3$ is independently selected from the group consisting of lower alkyls and aryl; and
n is an integer between 1 and 5; and

(b) subjecting the solution of step (a) to a temperature between 110 and 160 °C.

Step (a) corresponds to steps 001 and 002 and step (b), and step (c) corresponds to step 003 in Figure 8.

Preferably, $R^1$ and $R^2$ are $-CH_3$, the $R^3$-groups are $-CH_2\text{-}CH_3$, and n=3.

Preferably, in step (b), the solution of step (a) is subjected to a temperature between 110 and 160 °C for a period of time such that rate of growth of the nanostructures is significantly lower than the initial rate of growth, for example to a temperature of 125 °C for a period of 200 hours, or to a temperature of 140 °C for a period of 48 hours.

Optionally, the method further comprises a step of (c) allowing the solution of step (b) to cool to ambient temperature.

When the nanostructures have been formed by the linking of a multitude of monomers into a polymeric network, the residues of the monomers are designated monomer residues. They still retain the underlying covalent bond pattern of the original free monomers but the formation of the linkages modifies the groups directly involved in said linkages.

In the resulting nanostructures, the monomer residues are incorporated in the polymeric framework by means of -O-Si bonds, wherein the silicon atom is a silicon atom in the above structure.

The chelating groups comprise geminal bisphosphonate groups, i.e. two phosphonate groups attached to the

same carbon atom.

It is conceivable to mix two, three or several different polymer frameworks produced by the present method in any chemically compatible monomer combination, either by mixing the monomers prior to polymerization, or by grafting one polymer to another.

The degree of polymerization (average number of monomer residues), or alternatively, the molecular weight, of the nanostructures produced by the method disclosed herein may be precisely controlled to yield products of the desired size by manipulating the process parameters. Examples of such parameters are described in Example 1. It is less useful to describe size by degree of polymerization by stating the molecular weight rather than the hydrodynamic diameter, but it is another way of conceptualizing the structures. The degree of polymerization is included not as limiting but rather as a reference. For example, for a nanostructure with a diameter of 17.5 nm derived from 1,7-bis-(triethoxysilyl)-4,4-bis-(dimethylphosphonato)-heptane through a hydrolytic condensation polymerization according to the method described herein, with a nanostructure density of 1.469 g/cm$^3$, the estimated degree of polymerization would be about 6400 monomers and the molecular weight about 2.5 MDa.

For impure starting materials, i.e. monomers having a purity of less than 80%, the size of the produced nanostructures continues to grow indefinitely, as evident by the formation of precipitates, giving a broader size distribution (Example 10).

Alternatively, monomers of a certain purity, such as having a purity of more than 80%, preferably more than 85%, more preferably more than 90%, even more preferably more than 95%, especially more than 96%, can be used as starting material.

A series of size selection steps may optionally be performed on the solution of nanostructures produced from such monomers to remove undesirably large or small entities to lower the dispersity. Such size selection steps are described in more detail below.

When the monomer silane is of sufficient purity, such as having a purity of more than 80%, preferably more than 85%, more preferably more than 90%, even more preferably more than 95%, especially more than 96%, the size of the produced nanostructures plateaus at a given size, the size depending on the reaction conditions. When this plateau has been reached, the degree of crosslinking is substantially as high as it can get and the phosphonate esters are essentially completely hydrolyzed or hydrolyzed to a large extent. This is important for the application of said nanostructures in nanomaterial-based radioisotope therapy and imaging products since the product of complete hydrolysis, the bis-phosphonic acid is the most chelating form of the phosphonates (see Example 5).

Preferably, the degree of purity of the silane monomer is 80% or higher, such as 85% or higher, more preferred the degree of purity of the silane monomer is 90% or higher, even more preferred, the degree of purity of the silane monomer is 95%, especially more than 96% or higher. Such a high degree of purity may be accomplished by the purification method described herein.

Bis-(trialkoxy-)silanes, i.e. silanes in which the $R^3$-groups are lower alkyls, carrying phosphonate groups have been found to be particularly useful for the formation of nanostructures according to the present disclosure.

Preferably, $R^1$ and $R^2$ are independently selected from the group consisting of lower alkyls, i.e. alkyls having 1-8 carbon atoms, more preferably selected from the group comprising, methyl, ethyl and propyl, even more preferred, $R^1$ and $R^2$ are methyl.

Preferably, the $R^3$-groups are independently selected from the group consisting of lower alkyls, i.e. alkyls having 1-8 carbon atoms, more preferably selected from the group comprising, methyl, ethyl and propyl, even more preferred, the $R^3$-groups are ethyl-groups.

Preferably, n=3.

It has been found that 1,7-bis-(triethoxysilyl)-4,4-bis-(dimethyl-phosphonato)-heptane is a suitable monomer, in particular when the purity is above 80%, or above 85%, or above 90%, or above 95%, or above 96% as measured by HPLC-ELSD. The high purity promotes predictability of the polymerization process. The high purity may be achieved by the purification method disclosed herein.

Preferably, at least 50%, such as at least 90% of the monomer residues are 1,7-bis-(triethoxysilyl)-4,4-bis-(dimethylphosphonato)-heptane.

The mixture of water and a lower alcohol may be a mixture of 5 to 50% (vol/vol), preferably 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol), of water in a lower alcohol, the lower alcohol having 1-8 carbon atoms.

Preferably, the lower alcohol is chosen from the group consisting of ethanol, 1- propanol, 2-propanol, or 1,2-propanediol, 1,3-propanediol and ethylene glycol or mixtures thereof.

In one embodiment, the mixture of water and a lower alcohol is a mixture of 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol), of water in ethanol.

In another embodiment, the mixture of water and a lower alcohol is a mixture of 5-25% (vol/vol), such as 5-22% (vol/vol), such as 6-20% (vol/vol), such as 7-18% (vol/vol), such as 8-15% (vol/vol), such as 9-12% (vol/vol), such as

10-11% (vol/vol), of water in 1-propanol.

In a further embodiment, the mixture of water and a lower alcohol is a mixture of 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol), of water in 2-propanol.

In yet another embodiment, the mixture of water and a lower alcohol is a mixture of 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol), of water in 1,2-propanediol.

In a further embodiment, the mixture of water and a lower alcohol is a mixture of 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol), of water in 1,3-propanediol.

In another embodiment, the mixture of water and a lower alcohol is a mixture of 5-25% (vol/vol), such as 5-22% (vol/vol), such as 6-20% (vol/vol), such as 7-18% (vol/vol), such as 8-15% (vol/vol), such as 9-12% (vol/vol), such as 10-11% (vol/vol), of water in ethylene glycol.

In one embodiment, the mixture of water and a lower alcohol is a mixture of 5-25% of water in ethylene glycol is used.

In a further embodiment, the mixture of water and a lower alcohol is a mixture of 18-22% of water in ethylene glycol is used.

In another embodiment, the mixture of water and a lower alcohol is a mixture of 9-11% of water in ethylene glycol is used.

A suitable solvent mixture is water/ethylene glycol in a suitable ratio such as from 50% to 95% ethylene glycol or from 80% to 95% ethylene glycol or from 88 to 92% ethylene glycol, i.e. 5-50% (vol/vol), such as 5-20% (vol/vol), such as 8-12% (vol/vol) of water in ethylene glycol.

The heating can occur either in a sealed, pressure resistant vessel or as reflux at atmospheric pressure.

When low boiling alcohols are used it is necessary to work with closed pressure resistant vessels to achieve the desired reaction temperature.

In step (b) the temperature may be between 110 to 160 °C for up to 10 days.

The temperature in step (b) may be 120 to 145 °C, such as 120 to 140 °C.

The minimum time period in step (b) is dependent on the temperature, such that a temperature of 125 °C requires a time period of heating of 195 hours or more and a temperature of 140 °C requires a time period of heating of 48 hours or more. Preferably, longer time periods than the minimum time periods are used.

The minimum time period in step (b) is characterized by that the growth rate of the nanostructures after the minimum time period is significantly lower than the growth rate in the beginning of the reaction.

It is obvious to one skilled in the art that from the growth rate at the end of step (b) and the size of the nanostructures at the end of step (b) one can determine if growth rate is significantly lower than the initial growth rate without first having measured the initial growth rate.

In one embodiment, the growth rate of the nanostructures at the end of the step (b) is much lower than at the beginning of step (b), such as more than 10 times slower, or more than 20 times slower, or even more than 30 times slower.

In another embodiment, growth rate of the nanostructures at the end of the step (b) is essentially zero.

In a further embodiment, the conditions of step (b) are a temperature of 125 °C and a duration from 200 to 500 hours.

In yet another embodiment, the conditions of step (b) are a temperature of 140 °C and a duration from 40 to 200 hours.

Notably, the silane starting materials are sensitive to moisture and if exposed to moisture, e.g. by exposure to the atmosphere, or glassware, they will gradually form oligomers. The presence of such oligomers causes variability (Example 9) of the reaction rate and final size and size distribution of the nanostructures produced. Thus, measures should be taken to avoid exposing the monomers to moisture. Such measures are well known to the skilled person.

High-quality nanostructures of a given size between 13 and 90 nm, such as between 13 and 50 nm, or between 14 and 25 nm, or between 15 and 22 nm, or between 16 and 20 nm may be produced by the method disclosed above. It is imaginable that the method disclosed above can be used to produce nanostructures of smaller or larger size.

Examples of conditions are described in Example 1. An industrial and economic advantage of the process described here is that only environmentally friendly, and rather non-toxic solvents are in use.

In a specific embodiment, the monomer concentration is 25-85 mM, the solvent mixture is 20% (vol/vol) water in ethylene glycol, the reflux temperature is 125 °C and the reaction time is more than 250 hours.

In another specific embodiment, the monomer is 1,7-bis-(triethoxysilyl)-4,4-bis-(dimethylphosphonato)-heptane, the monomer concentration is 25-85 mM, the solvent mixture is 80% (vol/vol) ethylene glycol in water, the reflux temperature is 125 °C and the reaction time is more than 250 hours.

In another specific embodiment, the monomer is 1,7-bis-(triethoxysilyl)-4,4-bis-(dimethylphosphonato)-heptane, the monomer concentration is 30-40 mM, the solvent mixture is 10% (vol/vol) water in ethylene glycol, the reflux

temperature is 140 °C and the reflux time is about 48 hours.

In another specific embodiment, the monomer is 1,7-bis-(triethoxysilyl)-4,4-bis-(dimethylphosphonato)-heptane, the monomer concentration is 34-36 mM, the solvent mixture is 10% (vol/vol) water in ethylene glycol, the reflux temperature is 140 °C and the reflux time is about 48 hours.

In a further specific embodiment, the monomer is 1,7-bis-(triethoxysilyl)-4,4-bis-(dimethylphosphonato)-heptane, the monomer concentration is 34-36 mM, the solvent mixture is 10% (vol/vol) water in ethylene glycol, the reflux temperature is 140 °C and the reflux time is longer than 48 hours.

Optionally, a series of size selection steps may be performed on the solution of nanostructures to remove undesirably large (004) or small (005) entities to lower the dispersity. The steps 004 and 005 of Figure 8 can be performed in any order and repeated any number of times in any order. Starting materials and solvent residues from the reaction mixture can also be removed at this stage.

Importantly, the size selection steps may not be required if the starting material (i.e. the monomer) has a purity of more than 80%.

Ultrafiltration is a preferred method of size selection, especially when used in the form which is commonly called tangential flow filtration. Other ultrafiltration methods such as spin filters or dialysis can also be used although they are less scalable.

It is preferred to remove undesirably large nanostructures and/or aggregates by passing the solution through a filter with relatively large pores (004). A preferred nominal cut-off value for such filters is 0.2 μm. Alternatively, the nominal cut-off value for such filters are 1000 kDa, 500 kDa, or 300 kDa.

In step (005) the desired material can be collected on a filter with smaller pore size. Preferred pore sizes for step 005, given as nominal cut-off values, are 300 kDa, 100 kDa, 50 kDa, 30 kDa, or 10 kDa, with the proviso that when a 300 kDa filter is used in step 005, the filter used in step 004 must have larger pores. It should be noted that the nanostructures in this state have reactive surfaces and during any size-selection step, excessive up-concentration should be avoided or the nanostructures may aggregate irreversibly. Such up-concentration may be avoided by ensuring that the volume is kept essentially constant during the filtration process.

The material may be washed with several portions of a solvent, such as water, in step 005 to further remove unreacted monomers or unwanted solvent residues from step 001, 002 or 003.

Nanostructures of the desired size range may also be selected by size exclusion chromatography (also called gel filtration).

*Use* of *globular nanostructures according to the present disclosure*

Nanostructures according to the present disclosure may be used as precursors for other materials or as intermediates in the production of other materials.

***Use as an intermediate in the production of coated nanostructures***

One such application is as an intermediate in the production of a coated nanostructure. The coating may be a coating of polyethylene glycol (PEG). Typically, the average hydrodynamic diameter of the final coated nanostructures may be between 18 and 100 nm, or between 20 and 50 nm, or between 25 and 35 nm.

A coated nanostructure may be used for imaging or radiotherapy.

Specifically, such coated nanostructures may be used as an intravenous imaging agent and/or radiotherapeutic agent. Preferably, the coated nanostructures are encompassed in a composition suitable for such application, such as a liquid composition.

In one embodiment, a nanostructure according to the present disclosure is used as an intermediate to produce a pharmaceutical comprising PEG (polyethylene glycol)-coated nanostructures suitable for carrying a radioisotope for imaging and/or radiotherapy.

Example 6 describes how coated nanostructures according to the present disclosure having properties compatible with a pharmacological product. Such nanostructures may be used in tumor imaging and therapy. When used, the radioactive isotopes are incorporated into the nanostructures.

The radioactive isotopes may be incorporated into the nanostructures before delivery to the clinic or just before injection into the patient.

***Use as an intermediate in the production of a pharmaceutical product***

Another application is the use of a nanostructure according to the present disclosure as an intermediate in the production of a pharmaceutical product. Such a pharmaceutical product may be used in imaging and/or in radiotherapy.

Preferably, the nanostructures are coated nanostructures as described above.

When used, the radioactive isotopes are incorporated into the nanostructures. The radioactive isotopes may be incorporated into the nanostructures before delivery to the clinic or just before injection into the patient.

***Use as a carrier of a radioactive isotope***

Nanostructures according to the present disclosure may thus be used as carriers of radioactive isotopes.

***EXAMPLES***

Examples of different embodiments of the present disclosure will be described below.

***General experimental conditions***

Materials, reagents and solvents were obtained from commercial sources and were used without further purification unless otherwise noted. Solvents were of reagent grade or similar if not otherwise noted.

1,7-bis-(triethoxysilyl)-4,4-bis-(dimethylphosphonato)-heptane was prepared according to published procedures (WO2013041623A1, Example 3).

HPLC of 1,7-bis-(triethoxysilyl)-4,4-bis-(dimethylphosphonato)-heptane was performed on a Hewlett Packard Series 1100 equipped with an Agilent Poroshell 120 EC-C18 3.0 x 50 mm column eluting at 0.7 ml/min with an oven temperature of 40 °C, a DAD detector recording at 220 nm and an ELSD detector (ELSD settings: 36 °C, 1.4 l $N_2$/min, gain=8, impactor on).

HPLC of all other compounds was performed on a Hewlett Packard Series 1100 equipped with an Agilent Poroshell 120 EC-C18 4.6 x 50 mm column, a DAD detector recording at 220 nm and an ELSD detector (ELSD settings: 40 °C, 1.4 l N2/min, gain=4, impactor on), using a nonlinear gradient starting at 43% acetonitrile in water, at 1 ml/min with an oven temperature of 40 °C.

SEC was performed on a Younglin Instrument YL9100 equipped with an Agilent Bio SEC-5 1000 Å column eluting at 1.2 ml/min at ambient temperature a DAD detector recording at 220 nm, 280 nm and 560 nm, and an ELSD detector (ELSD settings: 60 °C, 1.2 l $N_2$/min, gain=4).

DLS was measured using a Malvern Instruments Zetasizer Nano ZEN3600 and processed using the general process setting in the Zetasizer software.

***Example 1 - Synthesis of nanostructures***

Three different variants ("Method 1", "Method 2" and "Method 3") of the method of producing globular nanostructures according to the present disclosure are disclosed below. The process parameters, e.g. the monomer concentration and purity, the nature of the solvent as well as the reaction time vary between these different variants.

Method 1 describes a method run at atmospheric pressure, suitable for large scale syntheses of nanostructures. Method 2 describes a method in closed vessels at pressure higher than one atmosphere, suitable for small scale syntheses of nanostructures. Method 3 is a reference method, not within the scope of the present disclosure, where the nanostructures are collected before the nanostructures have achieved a stable size and before the phosphonate esters and alkoxy silanes are fully hydrolyzed.

*Example 1A, representative example of "Method 1" (Long boiled)*

A 1-liter jacketed reactor was equipped with a mechanical stirrer, a temperature probe, and a reflux condenser topped with a connection to a vacuum-nitrogen manifold. The reactor was charged with 16.3 g of 1,7-bis(triethoxysilyl)-4,4-bis(dimethylphosphonato)heptane of a purity of 97% and 748.3 g of 90.0% (vol/vol) aqueous ethylene glycol. The solution was degassed. The mantle temperature was increased to 146 °C over a period of 25 min with stirring, during which the solution became clear. After 25 minutes at the set mantle temperature, a gentle reflux was obtained. The reaction mixture was kept at reflux for 47.5 h before being cooled down to 20 °C. Sampled for DLS after 45 and 47 hours of reflux, giving mean diameters of 18.1 nm, and 18.2 nm, respectively. Mean diameter 18.2 nm. $Đ_d$ = 1.20. [P](ICP-OES)= 69 mM, [Si](ICP-OES)= 74 mM.

*Example 1B, representative example of "Method 2" (Long boiled)*

A 250 ml bomb vial was charged with 7.91 g of 1,7-bis(triethoxysilyl)-4,4-bis(dimethylphosphonato)heptane of a purity of 96% and 182.5 ml of 80% (vol/vol) aqueous ethylene glycol. The solution was degassed and put under an atmosphere of nitrogen. The bomb vial was closed off and heated to 125 °C in an oil bath for 264 hours. The solution

was filtered through a glass fiber filter. Mean diameter 17.5 nm. $Đ_d$ = 1.26. [P](ICP-OES) = 136 mM, [Si](ICP-OES)= 154 mM.

*Example 1C, representative example of "Method 3" (Short boiled) - reference method not within the scope of the present disclosure*

In a round bottom flask, 4.53 g of 1,7-bis(triethoxysilyl)-4,4-bis(dimethyl-phosphonato)heptane of an estimated purity of 90% was mixed with 667 µl of a 23 mg/ml solution of rhodamine isothiocyanate in ethylene glycol, 71 ml ethylene glycol and 17.9 ml water. The solution was degassed and 12 ml portions put into closed reaction vials. The reaction vials were heated in a preheated 125 °C in an oil bath for 43 hours. Mean diameter 15.5 nm. $Đ_d$ = 1.33. [P](ICP-OES)= 164 mM, [Si](ICP-OES)= 165 mM.

*Examples 1D to 1Z*

Further experiments, in which the process parameters of the three variants described above have been varied, have also been performed.

The results of Experiments 1A-1Z are shown in Table 1.

***Table 1.*** *Results of using different reaction conditions in a method according to the present disclosure for producing nanostructures.*
*"Monomer" = 1,7-bis(triethoxysilyl)-4,4-bis (dimethylphosphonato)heptane.*
*Examples marked with * are reference examples outside the scope of the current disclosure*

| **Example** | **Method** | **Monomer Concentration (mM)** | **Purity of monomer (%)** | **Concentration of ethylene glycol in solvent mixture (% vol/vol)** | **Reaction time (hours)** | **Size of product nanostructures (nm)** | **Dispersity of product, Đ$_d$** |
|---|---|---|---|---|---|---|---|
| 1A | 1 | 35 | 97 | 90.0 | 49 | 18.2 | 1.20 |
| 1B | 2 | 65 | 96 | 80 | 264 | 17.5 | 1.26 |
| 1C* | 3 | 75 | 90 | 80 | 43 | 15.5 | 1.33 |
| 1D* | 2 | 25 | 96 | 80 | 402 | 11.4 | 1.22 |
| 1E* | 2 | 28 | 96 | 80 | 402 | 12.3 | 1.22 |
| 1F* | 2 | 30 | 96 | 80 | 402 | 12.2 | 1.25 |
| 1G | 2 | 34 | 96 | 80 | 402 | 13.5 | 1.24 |
| 1H | 2 | 37 | 96 | 80 | 402 | 14.3 | 1.24 |
| 1I | 2 | 41 | 96 | 80 | 402 | 15.4 | 1.22 |
| 1J | 2 | 45 | 96 | 80 | 402 | 15.3 | 1.28 |
| 1K | 2 | 50 | 96 | 80 | 402 | 16.1 | 1.30 |

| | | | | | | | |
|---|---|---|---|---|---|---|---|
| 1L | 2 | 55 | 96 | 80 | 402 | 17.4 | 1.29 |
| 1M | 2 | 61 | 96 | 80 | 402 | 17.7 | 1.31 |
| 1N | 2 | 67 | 96 | 80 | 402 | 20.5 | 1.27 |
| 1O | 2 | 75 | 96 | 80 | 402 | 22.6 | 1.28 |
| 1P | 2 | 82 | 96 | 80 | 402 | 23.9 | 1.30 |
| 1Q* | 1 | 15 | 97 | 90 | 135 | 12.5 | 1.20 |
| 1R | 1 | 20 | 97 | 90 | 135 | 14.1 | 1.25 |
| 1S | 1 | 25 | 97 | 90 | 135 | 15.9 | 1.18 |
| 1T | 1 | 30 | 97 | 90 | 135 | 16.2 | 1.20 |
| 1U | 1 | 40 | 97 | 90 | 135 | 20.1 | 1.19 |
| 1V | 1 | 50 | 97 | 90 | 135 | 23.4 | 1.24 |
| 1W | 1 | 65 | 97 | 90 | 135 | 24.7 | 1.24 |
| 1X | 1 | 35 | 96 | 89.0 | 48 | 16.7 | 1.22 |
| 1Y | 1 | 35 | 96 | 90.0 | 48 | 18.3 | 1.21 |
| 1Z | 1 | 35 | 96 | 91.0 | 48 | 18.9 | 1.21 |

As can be seen in Table 1, the hydrodynamic diameter of the nanostructures can be controlled by controlling the concentration of monomer at the start of the synthesis, as performed by Method 1 (1A, 1Q-1W) or by method 2 (1B, 1D-P). Additionally, the hydrodynamic diameter of the nanostructures can be controlled by controlling the concentration water in the reaction medium (1X-1Z).

***Example 2 - Purification of 1,7-bis(triethoxysilyl)-4,4-bis(dimethyl-phosphonato)heptane***

A dry three-necked 2-liter round bottom flask was charged with 107 g of 1,7-bis(triethoxysilyl)-4,4-bis(dimethylphosphonato)heptane of an estimated purity of 54%. Anhydrous acetonitrile (1070 ml) was added under vigorous stirring. After 30 minutes of vigorous stirring the stirring speed was lowered to ca 40 RPM and left overnight. The solution was cannula filtered through a 0.2 μm PTFE filter and the solvent removed in vacuo resulting in 98.9 g of a pale-yellow oil.

The resulting oil was mixed with heptane (1000 ml), resulting in the immediate formation of a sticky precipitate. The supernant was decanted into a fresh flask, the precipitate washed with heptane and the washings added to the supernant. The solution was gently stirred overnight before the clear supernant was decanted. The solution was dried over freshly activated molecular sieves by gentle agitation using a shaker table for 8 days before it was filtered first through a glass fiber filter and finally cannula filtered through a 0.2 μm PTFE filter. The solvent was removed in vacuo to yield 74 g of a clear oil.

61.5 g of the resulting oil was subjected to falling film distillation utilizing a home-built distillation apparatus similar to the one sold by Sigma Aldrich (e.g. Sigma Aldrich catalog number Z156604 as offered by Merck KGaA, Darmstadt, Germany) at a pressure of 1.3 mbar with the heating element heated to 160 °C and an addition speed of approximately 10 ml/h, resulting in 44 g of concentrate. The concentrate was determined to be 97% pure 1,7-bis(triethoxysilyl)-4,4-bis(dimethylphosphonato)heptane by HPLC-ELSD.

***Example 3*** - ***Failed attempt at vacuum distillation of 1,7-bis-(triethoxysilyl)-4,4-bis-(dimethylphosphonato)-heptane*** - *reference method not within the scope of the present disclosure*

In a two necked 50 ml round bottom flask equipped with a distillation head, a Liebig condenser and a receiving flask, was added 4.6 g of crude 1,7-bis-(triethoxysilyl)-4,4-bis-(dimethylphosphonato)-heptane. Oil-pump vacuum (0.1 mbar) was applied and the distillation flask was heated gradually in an oil-bath from 40°C to a temperature of 210 °C.

The maximum inner temperature reached was 142 °C. No product was collected in the receiving flask and the residue in the distillation flask was very dark brown and not suitable for use as starting material for nanostructures.

Thus, it has been demonstrated that crude 1,7-bis-(triethoxysilyl)-4,4-bis-(dimethylphosphonato)-heptane cannot be purified using conventional vacuum distillation.

***Example 4** - **Chelating strength of coated nanostructures of various size determined by EDTA Competitive chelation test***

Nanostructures were synthesized by methods according to example 1 resulting in nanostructures of 7.1, 12.5, 17.5, and 23 nm for test items 4A, 4B, 4C, and 4D, respectively.

The test items were coated by a procedure similar to the one employed in example 6 below and loaded with metal ions ($M^{3+}$), such as yttrium (Y) or lutetium (Lu). The metal ions loaded nanostructures were then diluted with 50 mM Tris buffer, pH 7.4 to a concentration of 0.2 mM yttrium or lutetium. A 2 mM Ethylenediaminetetraacetic acid (EDTA) solution in 50 mM Tris buffer pH 7.5 was prepared for a test of using 10 molar eq. excess of EDTA to nanostructure-bound metal ions, or a 1 mM EDTA solution for a test of using 5 molar eqv (equivalent) excess of EDTA to nanostructure-bound metal ions. 280 μL of diluted metal ions loaded nanostructure and 280 μL prepared EDTA solution were mixed and allowed to stand at room temperature for 1 h (1hRT), or for 24 h at 37 °C (24h37C). After incubation, 50 μl of the mixture was removed and labelled XXX-pre (XXX = id of choice, pre = pre filtration). The remaining mixture solution was placed into an 0.5 ml Amicon 3 kDa spin filter and centrifuged for 15 min at 12000 x g). The permeate was labelled XXX-post (post = post filtration). Metal ions concentrations in samples xxx-pre and xxx-post were determined by ICP-OES. The percentage of leftover nanostructure - bound metal ions were calculated using the equation below, termed metal ion stability%. The resulted value is referred to as chelating strength of the nanostructure, meaning that the larger the value is, the stronger chelating strength the nanostructure has.

$$Metal\ ion\ stability\ (\%) = 100 - \left(\frac{[M^{3+}]_{xxx-post}}{[M^{3+}]_{xxx-pre}} \times 100\right)$$

***Table** 2. Chelating strength of coated nanostructures. "core" refers to the uncoated nanostructure prior to coating; 1hRT denotes incubation for 1 h at room temperature; 24h37C denotes incubation for 24 h at 37 °C.*

| **Test item** | **Mean core size (nm)** | **Metal ions ($M^{3+}$)** | **EDTA eqv** | **Metal ions stability (%) (1hRT)** | **Metal ions stability% (24h37C)** |
|---|---|---|---|---|---|
| 4A* | 7.1 | Y | 5 | 93 | NA |
| 4B* | 12.5 | Lu | 10 | 99 | 91.5 |
| 4C | 17.5 | Lu | 10 | 99 | 97.3 |
| 4D | 23 | Lu | 10 | 99 | 98.3 |

As can be seen in Table 2, coated nanostructures where the chelating central part is smaller than 13 nm has significantly weaker chelating strength, as compared to coated nanostructures where the chelating central part is larger than 13 nm.

***Example 5** - **Chelating strength comparison of short and long boiled nanostructures***

Nanostructures were synthesized by procedures similar to example 1B and 1C, resulting in nanostructures of 12.4, 12.5, 17.8, and 17.5 nm for test items 5A, 5B, 5C, and 5D, respectively. Test items 5B and 5D were synthesized by procedures similar to example 1B and test items 5A and 5C were synthesized by procedures similar to example 1C.

The test items were coated by a procedure similar to the one employed in example 6 below and loaded with metal ions ($M^{3+}$), such as yttrium (Y) or lutetium (Lu). The metal ions loaded nanostructures were then diluted with 50 mM Tris buffer (pH 7.4) to a concentration of 0.2 mM yttrium or luthetium. A 2 mM Ethylenediaminetetraacetic acid (EDTA) solution in 50 mM Tris buffer, pH 7.5 was prepared for a test of using 10 molar eqv excess of EDTA to nanostructure-bound metal ions, or a 1 mM EDTA solution for a test of using 5 molar eq. excess of EDTA to nanostructure-bound metal ions. The larger excess of EDTA, the harsher the test is. 280 μL diluted metal ions loaded nanostructures and 280 μL of the EDTA solution were mixed and incubated at room temperature for 1 hour. After incubation, 50 μl of

the mixture was removed and labelled XXX-pre (XXX = id of choice, pre = pre filtration). The remaining mixture solution was placed into an 0.5 ml Amicon 3 kDa spin filter and centrifuged for 15 min at 13.4 krpm (= 12000 x g). The permeate was labelled XXX-post (post = post filtration). Metal ion concentrations in samples xxx-pre and xxx-post were determined by ICP-OES. The percentage of leftover nanostructure -bound metal ions were calculated using the equation below, termed metal ions stability%. The resulted value is a measure of the chelating strength of the nanostructure, meaning that the larger the value is, the stronger chelating strength the nanostructure has.

$$Metal\ ion\ stability\ (\%) = 100 - \left(\frac{[M^{3+}]_{xxx-post}}{[M^{3+}]_{xxx-pre}} \times 100\right)$$

***Table** 3. Chelating strength of coated nanostructures. "core" refers to the uncoated nanostructure. Test items 5A, 5B, and 5C, marked with an * are reference items not within the scope of the present disclosure.*

| Test item | Synthesis method | Mean core size (nm) | Metal ions ($M^{3+}$) | EDTA eqv | Metal ion stability (%) |
|---|---|---|---|---|---|
| 5A* | Short (1C) | 12.4 | Y | 5 | 94 |
| 5B* | Long (1B) | 12.5 | Lu | 10 | 99 |
| 5C* | Short (1C) | 17.8 | Y | 5 | 97.5 |
| 5D | Long (1B) | 17.5 | Lu | 10 | 99 |

Table 3 shows that long boiled nanostructures loaded with metal ions, synthesized by method 1B or 1A, have better metal ion stability compared with the same size nanostructures but made by the short boiling method, 1C. This demonstrates the superior chelating strength of nanostructures of the present disclosure.

***Example 6** - **Biodistribution study of coated nanostructures in a tumor-bearing mouse model***

*Representative preparation of test items: Test item C*

Nanostructures were synthesized by procedures similar to example 1B using a 95 mM solution of 1,7-bis(triethoxysilyl)-4,4-bis(dimethylphosphonato)-heptane resulting in nanostructures with a diameter of 23.0 nm. To prepare coated nanostructures, a 10 ml sample of the resulting nanostructure solution (nominally 0.95 mmol 1,7-bis(triethoxysilyl)-4,4-bis(dimethylphosphonato)heptane) was charged in a vial and heated to 100 °C in an oil bath. After 20 minutes 809 mg of bis(triethoxysilyl)methane (2.38 mmol, 2.5 equivalents to 1,7-bis(triethoxysilyl)-4,4-bis(dimethylphosphonato)heptane) was added with vigorous stirring. The heating was maintained for 4 hours with more moderate stirring. Mean diameter 25.7 nm.

A 1.025 ml sample of the resulting nanostructure solution (nominally 0.097 mmol 1,7-bis(triethoxysilyl)-4,4-bis(dimethylphosphonato)heptane) was diluted to 7.5 ml with 80% (vol/vol) aqueous ethylene glycol in a 2-necked round bottom flask with the vertical neck equipped with a reflux condenser topped connection to a vacuum-nitrogen manifold and the side neck stoppered with a septum. The solution was heated for 10 minutes in a preheated 110 °C oil bath before 200 μl of a 50% (w/w) solution of 1,7-bis(triethoxysilyl)-4,4-bis(ω-methyl-(ethyleneoxy)45-methyl)heptane in anhydrous dioxane heated to 40 °C was added. A further 805 μl of the warm 1,7-bis(triethoxysilyl)-4,4-bis(ω-methyl-(ethyleneoxy)45-methyl)heptane solution was added by syringe pump at a speed of 43 μl/hour. After a total of 48 hours the solution was allowed to cool to ambient temperature.

The resulting nanostructure solutions from two identical batches were combined and diluted to 50 ml with water and filtered through a 0.2 μm polyethersulfone (PES) syringe filter. The resulting solution was diluted to 800 ml with water and concentrated to approximately 20 ml using a tangential flow filtration setup with a 300 kD nominal cut off. The dilution - concentration procedure was repeated for a total of five times before the solution was further concentrated to 11.3 ml on a spin filter with a 300 kD nominal cut off. Mean diameter = 35.3 nm. [P](ICP-OES) = 28 mM.

A 4 ml sample of the resulting nanostructure solution (112 μmole P) was treated with 228 μl of a 19.7 mM $LuCl_3$ solution (4.5 μmole Lu) and heated to 60 °C for 1 hour before 1.269 ml of 1 M Tris buffer (pH=7.47) was added. After a further 2 hours at 60 °C the solution was filtered through a 0.2 μm PES syringe filter.

A 3.47 ml sample of the resulting nanostructure solution was mixed with 3.80 ml saline solution and 140 μl of a 99 mM $CaCl_2$ solution before pH was adjusted to 7.2 with 12 μl of a 1 M NaOH solution. The solution was diluted to

a total volume of 7.5 ml with saline before the solution was filtered through a 0.2 μm PES syringe filter.

Mean size (DLS) = 36.0 nm. [P](ICP-OES) = 9.9 mM, [Si](ICP-OES) = 46 mM, [Lu](ICP-OES) = 0.39 mM

*Test items A and B*

Test item A and B were prepared similarly starting from 37 mM and 65 mM solutions of 1,7-bis(triethoxysilyl)-4,4-bis(dimethylphosphonato)heptane, respectively.

Test item A: Mean size (DLS) = 21.3 nm. [P](ICP-OES) = 8.9 mM, [Si](ICP-OES) = 48 mM, [Lu](ICP-OES) = 0.39 mM
Test item B: Mean size (DLS) = 27.7 nm. [P](ICP-OES) = 8.7 mM, [Si](ICP-OES) = 41 mM, [Lu](ICP-OES) = 0.39 mM

*Biodistribution*

The tissue distribution of the test items after intravenous injection in a tumor-bearing mouse was investigated. Injection test items were analyzed by ICP-OES for lutetium, silicon and phosphorus content. The obtained values for lutetium represent the total amount (i.e. 100%) of lutetium injected. Each test item was administered intravenously at 2 μmol Lu/kg and 5 ml/kg. Animals were divided into 4 groups per test item (n=5 mice/group) and groups were sacrificed at 6 h, 24 h, 48 h and 168 h post-injection. Three animals were used as control animals and were not dosed. Blood samples were taken at the end of the study period and plasma was prepared by removal of blood cells. After termination, organs were harvested (liver, tumor and femoral muscle). Plasma samples and digested tissue samples were analyzed by ICP-OES for lutetium (shown in Table 4) and silicon (data not shown) content. Table 4 shows the distribution of the injected lutetium at different periods of time after injection of the test items.

***Table 4.*** *% of the injected dose (% ID) Lu after 6 h, 24 h, 48 h and 168 h. "N.M." = not measured.*

| Organ | Test item | 6 h | 24 h | 48 h | 168 h |
|---|---|---|---|---|---|
| Plasma | A | 80.2 ± 6.5 | 54.7 ± 3.0 | 24.3 ± 5.0 | N.M. |
| | B | 81.0 ± 2.5 | 51.7 ± 4.9 | 23.4 ± 2.3 | N.M. |
| | C | 82.2 ± 4.5 | 41.7 ± 4.5 | 18.6 ± 2.4 | N.M. |
| Liver | A | 8.9 ± 1.0 | 16.5 ± 1.7 | 19.8 ± 2.7 | 23.0 ± 3.1 |
| | B | 7.7 ± 1.1 | 13.6 ± 1.4 | 15.7 ± 3.3 | 22.9 ± 1.8 |
| | C | 8.8 ± 0.6 | 16.1 ± 1.0 | 21.6 ± 1.8 | 25.7 ± 2.3 |
| Tumor | A | 0.8 ± 0.3 | 2.4 ± 0.6 | 2.5 ± 1.5 | 3.3 ± 1.3 |
| | B | 1.0 ± 0.4 | 2.1 ± 0.5 | 2.6 ± 1.1 | 4.2 ± 1.8 |
| | C | 1.0 ± 0.4 | 2.5 ± 1.0 | 2.5 ± 1.1 | 5.0 ± 3.8 |
| Muscle | A | 4.5 ± 2.7 | 8.8 ± 2.4 | 7.6 ± 2.3 | 8.8 ± 2.0 |
| | B | 6.2 ± 0.9 | 10.0 ± 4.2 | 11.8 ± 2.7 | 16.0 ± 7.0 |
| | C | 7.1 ± 2.4 | 10.6 ± 3.3 | 6.2 ± 1.1 | 10.4 ± 2.7 |

As can be seen in Table 4, coated nanostructures according to the present disclosure are long-circulating, i.e. they have long plasma half-lives. Additionally, it can be seen that a significant fraction of the nanostructures distributes to the tumor. This demonstrates the suitability of nanostructures by the current disclosure for use in a pharmaceutical composition and for use in the imaging and/or therapy of cancer.

***Example 7*** - ***SEC chromatogram versus DLS***

A nanostructure according to example 1A that is 18.2 nm in average diameter based on DLS measurement, was also analyzed by size exclusion chromatography (SEC). The peak apex and size distribution were determined by comparing with reference proteins or protein complexes of known size.

Reference protein standards: Bovine serum albumin, 66 kDa, Thyroglobulin from bovine thyroid gland (Thyrobov), 667 kDa, and empty Cowpea Mosaic Virus (CPMV) VLPs, 4360 kDa.

The chromatogram in Fig. 6 shows that the nanostructure (thick line) give a bell shaped peak, and the apex of

the nanostructure peak is located close to Thyro-bov (19.4 nm by DLS). There is 95.6% of the nanostructure peak area lying between BSA (6.9 nm by DLS) and CPMV (29.1 nm by DLS), 1.6% of the nanostructure peak area is above CPMV, and 2.8% of the peak area is below BSA. The 2.8% portion is mainly residues of the starting monomer.

***Example 8 - NMR of short and long boiled nanostructure***

NMR spectra were recorded at 25°C on a Varian Unity Inova 500 MHz spectrometer equipped with a Z-spec DBG500-5EF 5 mm dual broadband gradient probe.

$^1$H spectra were recorded with an excitation pulse with a pulse width of 5.7 μs (corresponding to a 45° flip angle), an acquisition time of 1.0 s, a repetition delay of 5.0 s, with the collection of 4 transients and a spectral width of i) 200 kHz with 400k data points or ii) 12 kHz with 24k data points. Shimming was performed by gradient shimming on the solvent hydrogen signal. $^{31}$P NMR spectra were recorded with a pulse width of 4.9 μs, an acquisition time of 0.02 s, a repetition delay of 1.0 s, a spectral width of 506kHz, with the collection of 64 transients and 2k data points. Spectra were recorded both with and without proton decoupling.

Samples of nanostructures synthesized similarly to example 1C, "short boiled", and 1B, "long boiled", were transferred to $D_2O$ and analyzed by NMR.

The $^1$H NMR of short and long boiled nanostructures are shown in Fig 3. The long boiled give a considerably broader spectrum indicating a higher degree of cross-lining.

The $^{31}$P NMR spectra of short and long boiled nanostructures are shown in Fig 4. The long boiled give a single peak, indicating that there is only one type of phosphonate present, presumably the completely hydrolyzed as opposed to the short boiled sample which shows a more complex shape indicating partial hydrolysis.

**Example 9 - Synthesis of nanostructures with 1,7-bis(triethoxysilyl)-4,4-bis(dimethylphosphonato)heptane of low purity -** *reference method not within the scope of the present disclosure*

A 25 ml 3-necked round bottom flask with the central neck equipped with a reflux condenser topped with a connection to a vacuum-nitrogen manifold and the side necks closed with glass stoppers was charged with 720 mg of unpurified 1,7-bis(triethoxysilyl)-4,4-bis(dimethylphosphonato)heptane of a purity of 80% and 14.7 ml of 80% (vol/vol) aqueous ethylene glycol. The solution was degassed before being heated to a gentle reflux in an oil bath. After 44 hours of reflux this resulted in nanostructures with a diameter of 16.2 nm and a high dispersity ($Đ_d$ = 2.14).

Another reaction set up in the same manner using the same batch of unpurified 1,7-bis(triethoxysilyl)-4,4-bis(dimethylphosphonato)heptane gave after 45 hours of reflux nanostructures with a diameter of 86.6 nm and a high dispersity ($Đ_d$ = 1.95)

This demonstrates the unsuitability of impure 1,7-bis(triethoxysilyl)-4,4-bis(dimethylphosphonato)heptane for the synthesis of nanostructures according to the present disclosure.

***Example 10 - AFM images present a globular shape of nanostructures according to the present disclosure***

Nanostructures made according to example 1B having a mean particle size of 19.1 nm were subjected to atomic force microscopy (AFM) analysis.

AFM model: Fast Scan Asyst
Tip: SSS-NCHR-10 (2 nm), spring constant is 10-100 N/m
Imaging mode: Tapping mode with feedback gain of peak force instead of cantilever amplitude

A silicon wafer was cleaned by sonicating in acetone for 2 minutes and then blow dried with $N_2$ gas. Another 2 minutes' sonication was performed in 2-proponal and then the wafer was blow dried again with $N_2$ gas. The nanostructures were diluted in 10 mM aqueous ammonium bicarbonate [$(NH_4)HCO_3$] to 16 μM in phosphorus concentration, and then a drop of 10 μL was deposited on the cleaned silicon wafer. The drop on the wafer was dried on a 40 °C hotplate for 10 minutes and the nanoparticles were subjected to atomic force microscopy (AFM) analysis.

The result is shown in Fig 7, where it is seen that the individual particles fulfill the globular shape definition.

***Example 11 - Modeling of nanostructure composition from elemental analysis***

Nanostructures according to example 1A were dried by freeze-drying followed by further drying at 120 °C overnight before combustion elemental analysis was performed. The results were: C: 24.17%, H: 5.37%, Si: 15.42%, P: 14.81%. The modeling was done in Excel according to the following method: The suspected molecular components were added to their relative molar contributions. The main component 1,7-bis(triethoxysilyl)-4,4-bis(dimethylphosphonato)heptane is set to 1 and the molecular weight is set to the hypothetical, completely crosslinked, completely hydrolyzed version with a molecular weight of 362 g/mol. Water is then added to formally break crosslinks since Si-O-Si + $H_2O$ $\rightarrow$SiOH + HOSi. We know from NMR that we have about 4% by weight of ethylene glycol in the structure and that is added too. Then the elemental contribution of each molecular component is calculated and the percentage of each element is calculated and compared to the experimental values. The penalty function (sum of absolute differences between modelled and calculated values) of the deviations is minimized by adjusting the fractions. In this case it is only the water and ethylene glycol content to optimize. The best fit to combustion data is a molar ratio of 1: 0.39: 0.01 for 1,7-bis(triethoxysilyl)-4,4-bis(dimethylphosphonato)heptane, ethylene glycol and water, respectively, corresponding to 5.9 bonds between the monomers. This shows that the nanostructures have the intended composition with a very high number of bonds between the monomers.

***Example 12 - Determination of nanostructure powder density***

Freeze-dried nanostructures according to example 1A were suspended in a series of mixtures of heptane and dibromomethane with different densities. The suspensions were centrifuged at 13000 rpm for 5 mins and it was observed if the particles sediment, float, or suspend. Particles sediment if their density is higher than the heptane-dibromomethane mixture, particles float if their density is lower than the heptane-dibromomethane mixture, and particles stay in suspension if their density is similar to the respective heptane-dibromomethane mixture. The nanostructures sedimented in heptane-dibromomethane mixtures up to the density of 1.561 g/mL and floated in the solvent mixture with density of 1.736 g/mL. The mean value of these two densities 1.649 was hence estimated as the density of the nanostructures. The high density indicate that the phosphonate esters have hydrolyzed completely since bisphosphonic acids have considerably higher densities than the corresponding esters.

***Example 13 - Synthesis of 1,7-bis(triethoxysilyl)-4,4-bis(ω-methyl-(ethyleneoxy)45-methyl)heptane***

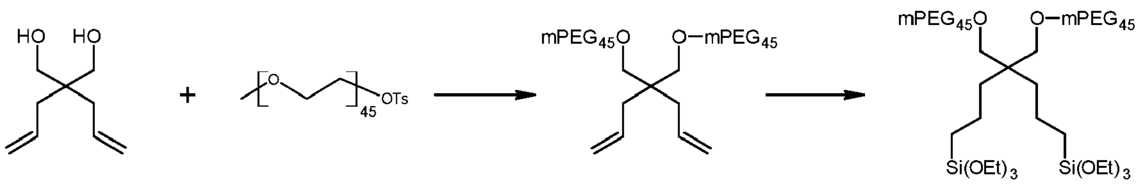

*Example 13a: synthesis of 4,4-bis(ω-methyl-(ethyleneoxy)45-methyl)-hepta-1,6-diene*

Diallyl propanediol (22 g, 0.1411 mol) was dissolved in anhydrous toluene (2.61 l) and cooled in an ice bath. When the inner temperature reached below 10 °C, NaH (23.7 g, 0.593 mol, 60 % in mineral oil, 4.2 eq) was added in three portions maintaining the temperature below 10 °C. The slurry was then stirred at room temperature for 60 minutes and then added to an azeotropically dried solution of m-$PEG_{45}$-OTs (1.071 kg, 0.9877 mmol, 3.5 eq) in anhydrous toluene (2.61 l) under $N_2$ at 0 °C. The reaction mixture was heated to reflux overnight and stirred under $N_2$. The reaction was monitored by HPLC and upon completion, the temperature was lowered to 15 °C and the reaction was quenched by a dropwise addition of $H_2O$ (70 ml). The pH of the crude reaction mixture was adjusted to between 5 and 7 with 1.0 M HCl (100 ml). The crude reaction mixture was split in two equal portions for practical reasons and the two portions extracted separately. Each half of the crude mixture was diluted with $H_2O$ (7.14 l). The temperature was increased to 60 °C and NaCl (540 g) was added. The mixture was then stirred for 45 minutes and extracted three times with EtOAc (2.1 l). To the remaining aqueous phase NaCl (200 g) was added and the mixture was again extracted three times with EtOAc (2.1 l). The last three extracted fractions had an acceptable product purity (HPLC) and were dried over $MgSO_4$, filtered through double GF/A filters and the solvent evaporated. The resulting residues were pooled and dissolved in $H_2O$ (2.0 l) and the pH was adjusted to pH 8 with an 0.8 M aqueous solution of $NaHCO_3$ (100 ml). The aqueous phase was extracted three times with DCM (500 mL). The organic phases were dried over $MgSO_4$, filtered and evaporated to obtain a white residue.

Consequently, the extraction process repeated for the other half of the crude mixture and the final products of both extractions were pooled with the product of another similarly sized batch synthesized in the same manner to obtain 4,4-bis(ω-methyl-(ethyleneoxy)$_{45}$-methyl)-hepta-1,6-diene (758 g, 66.36 % yield, 96.8 % purity (HPLC-ELSD)).

$^1H$ NMR (400 MHz, $CDCl_3$) δ 5.80 (m, 2H), 5.03 (m, 4H), 3.70-3.60 (s, 540H), 3.37 (s, 6H), 3.22 (s, 4H), 2.04 (d, 4H).

*Example 13b: synthesis of 1, 7-bis(triethoxysilyl)-4,4-bis(ω-methyl-(ethyleneoxy)$_{45}$-methyl)-heptane*

To an azeotropically dried solution of 4,4-bis(ω-methyl-(ethyleneoxy)$_{45}$-methyl)-hepta-1,6-diene (714 g, 0.172 mol) in toluene (5.5 l), triethoxysilane (1117 g, 6.88 mol, 40 eq) was added at 22 °C under nitrogen. Karstedt's catalyst (25.34 ml, 2 % in xylenes, 1.14 mmol, 0.0066 eq) was added in 1 mL portions using a syringe over 30 minutes which resulted in an exotherm of ≤ 2 °C. The reaction mixture was left stirring at 22 °C under nitrogen overnight.

The reaction was monitored by $^1$H-NMR for the disappearance of the olefinic protons. The solvent was then evaporated and excess silane was removed by co-evaporating with anhydrous toluene (2.5 l) for a total of four times. The residue was then redissolved in toluene (4.2 l) degassed with 3 cycles of vacuum/nitrogen and stirred with activated SIR-200 resin (175 g) for 3 days at 60 °C. The solution was filtered from the resin, the resin washed with toluene (3 x 2.8 l), the collected fractions were filtered through double GF/A filters, pooled and the solvent evaporated to obtain 1,7-bis(triethoxysilyl)-4,4-bis((ω-methyl-(ethyleneoxy)$_{45}$-methyl)-heptane as a white solid in quantitative yield (783.2 g, ≥99 %, 94.4 % purity (HPLC-ELSD)).

$^1$H NMR (400 MHz, $C_6D_6$) δ 3.71 (q, 12H), 3.70-3.40 (s, 400H), 3.52 (s, 4H), 3.35 (s, 6H), 1.68 (m, 4H), 1.59 (m, 4H), 1.23 (t, 18H), 0.79 (t, 4H).

## Claims

1. A plurality of globular nanostructures, wherein the plurality of globular nanostructures has a dispersity between 1 and 1.8; and

   wherein the nanostructures have a volume average hydrodynamic diameter of 13 nm to 90 nm;
   wherein each nanostructure comprises a polymer framework of monomer residues, wherein the average number of bonds from each monomer residue is in the range of from 3.0 up to but not including 6.0; wherein the linkages between the monomer residues are Si-O-Si; wherein each nanostructure comprises from 10% to 25% by weight of silicon; wherein at least 90% of the monomer residues have from 5 to 11 carbon atoms; wherein at least 90% of the monomer residues comprise two geminal chelating groups, each chelating group independently being a group according to Formula (I)

   $$-PO(OR^1)(OR^2) \qquad (I)$$

   wherein
   $R^1$ and $R^2$ are independently selected from the group consisting of a negative charge and H; and
   - denotes an internal bond in the monomer residue; and
   wherein the chelating groups according to Formula (I) constitute at least 90% of the chelating groups in the nanostructure.

2. A plurality of nanostructures according to claim 1, wherein at least 90% of the monomer residues are residues according to Formula (II):

   $$\{(OR^1)(OR^2)PO\}_2\text{-}(C)\{(CH_2)_nSi(OR^3)_3\}\{(CH_2)_nSi(OR^3)_3\} \qquad (II)$$

   wherein

   each $R^1$ and $R^2$ is independently selected from the group consisting of a negative charge and H;
   each $R^3$ is independently selected from the group consisting of a negative charge, H and a covalent bond to the polymeric framework; wherein at least 3 $R^3$ are bonds to the polymeric framework; and
   n is an integer between 1 and 5.

3. A plurality of nanostructures according to claim 2, wherein at least 4 of the $R^3$-groups are bonds to the polymeric framework.

4. A plurality of globular nanostructures according to claim 2 or 3, wherein n = 3.

5. A plurality of globular nanostructures according to any one of the preceding claims, wherein the nanostructures further comprise a coating, preferably wherein the coating comprises hydrophilic groups.

**6.** A pharmaceutical composition comprising a plurality of globular nanostructures according to claim 5.

**7.** A pharmaceutical composition for use in in the treatment of cancer and/or imaging, wherein the pharmaceutical composition comprises a plurality of globular nanostructures according to claim 5, wherein the globular nanostructures further comprise radioactive isotope.

**8.** A method for purifying 1,7-bis(triethoxysilyl)-4,4-bis(dimethoxyphosphonato)heptane, the method comprising the steps of

(a) providing a solution of impure 1,7-bis(triethoxysilyl)-4,4-bis(dimethoxyphosphonato)heptane in a polar aprotic solvent;
(b) separating the solution of step (a) from insoluble matter;
(c) concentrating the solution obtained in step (b), thereby providing a residue;
(d) dissolving the residue obtained in step (c) in a non-polar solvent;
(e) separating the solution obtained in step (d) from insoluble matter;
(f) removing water from the solution obtained in step (e);
(g) concentrating the solution obtained in step (f), resulting in a second residue;
(h) subjecting the residue obtained in step (g) to a short path, pass-through vacuum distillation; and
(i) collecting the pass-through fraction from step (h), comprising purified 1,7-bis(triethoxysilyl)-4,4-bis(dimethoxyphosphonato)heptane.

**9.** A method according to claim 8, wherein

- the polar aprotic solvent in step (a) is acetonitrile and the solution in step (a) has a concentration of impure 1,7-bis(triethoxysilyl)-4,4-bis(dimethoxy-phosphonato)heptane ranging from 25 g/l to 250 g/l; and/or
- the non-polar solvent in step (d) is a lower alkane, , and the solution in step (d) has a concentration of the residue obtained in step (c) ranging from 25 g/l to 250 g/l/l; and/or
- the short path, pass-through vacuum distillation in step (h) is performed at a temperature ranging from 150°C to 190 °Cand a pressure ranging from 0.1 mbar to 1 mbar.

**10.** Use of 1,7-bis(triethoxysilyl)-4,4-bis(dimethoxyphosphonato)-heptane purified according to claim 8 or 9 to produce a plurality of globular nanostructures according to anyone of claims 1 to 5.

**11.** A method for producing a plurality of globular nanostructures according to anyone of claims 1 to 4, comprising the steps of:

(a) providing a solution comprising monomers in a mixture of water and a lower alcohol, wherein the monomers are monomers according to Formula (II)

$$\{(OR^1)(OR^2)PO\}_2\text{-}(C)\{(CH_2)_nSi(OR^3)_3\}\{(CH_2)_nSi(OR^3)_3\} \qquad (II)$$

wherein

each $R^1$ and $R^2$ is independently selected from the group consisting of lower alkyls and aryl; and
each $R^3$ is independently selected from the group consisting of lower alkyls and aryl; and
n is an integer between 1 and 5; and

(b) subjecting the solution of step (a) to a temperature between 110 and 160°C.

**12.** A method according to claim 11, wherein the solution provided in step (a) is provided by dissolving monomers having a purity of more than 80%, in a mixture of water and a lower alcohol

**13.** A method according to claim 11 or 12, wherein the monomer in step (a) is 1,7-bis-(triethoxysilyl)-4,4-bis-(dimethylphosphonato)-heptane, and wherein the monomer concentration is 30-40 mM, the solvent mixture is 10% water in ethylene glycol, and, in step (b), the temperature is 140 °C and the heating time is 45 to 50 hours.

**14.** Use of a plurality of globular nanostructures of any of the claims 1 to 4 and/or the product of a method of any of claims 11 to 13 as an intermediate in the production of a plurality of globular coated nanostructures.

**15.** Use of the plurality of globular nanostructures according to any one of claims 1 to 5 and/or the product of a method of any of claims 11 to 13 as an intermediate in the production of a pharmaceutical product.

**16.** Use of a pharmaceutical composition according to claim 6 as a carrier of a radioactive isotope.

Fig. 1

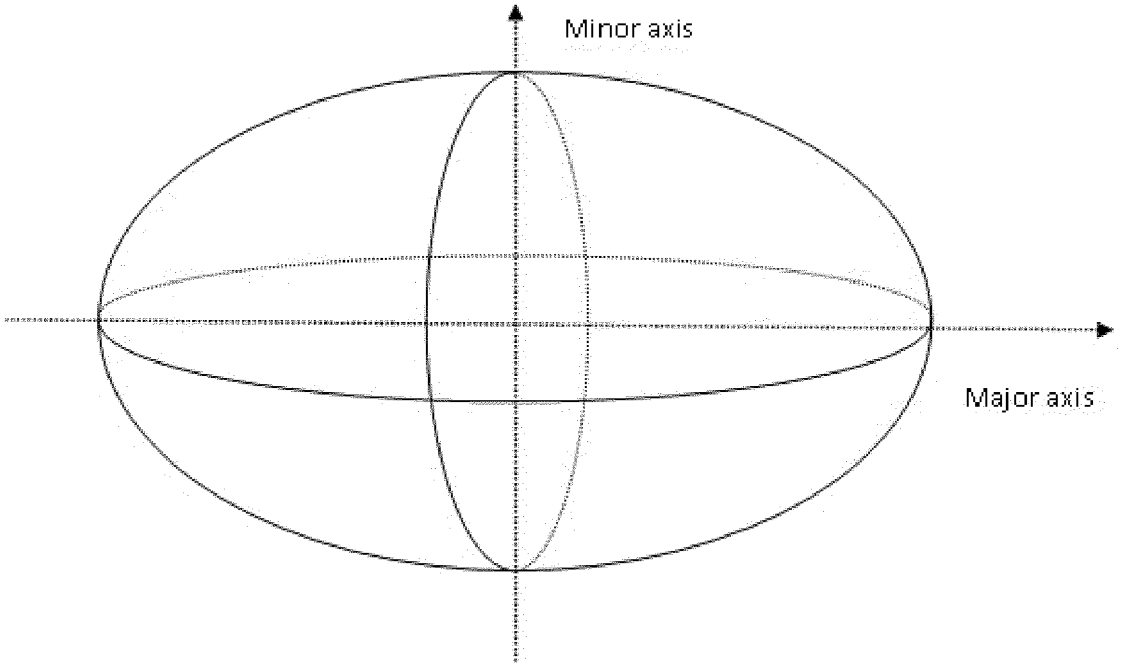


Fig. 2

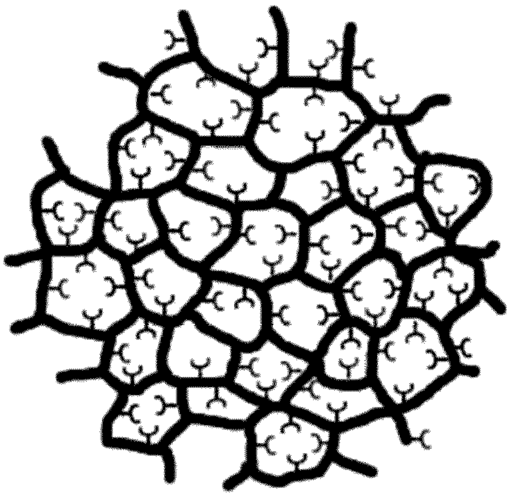

Fig. 3

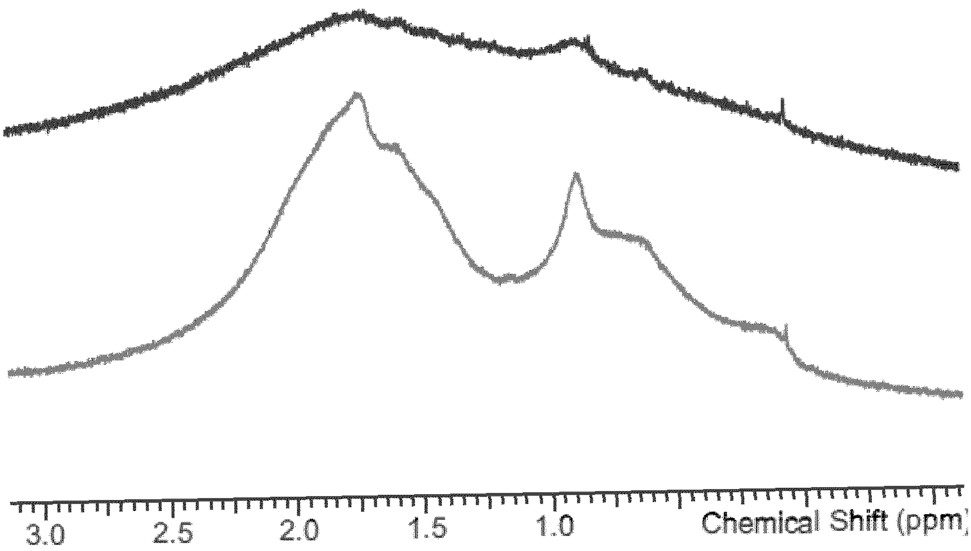

Fig. 4

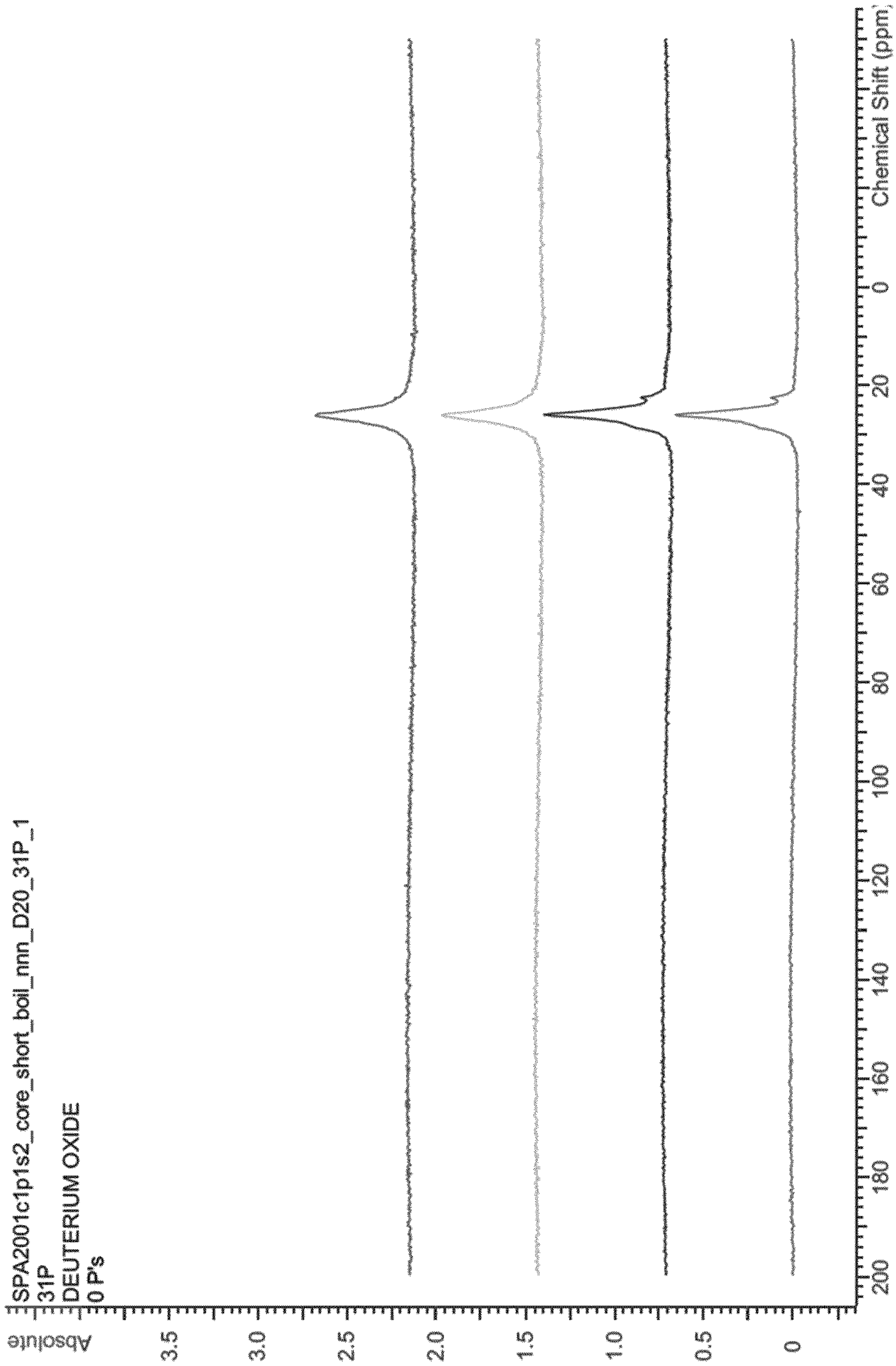
SPA2001c1p1s2_core_short_boil_nnn_D20_31P_1
31P
DEUTERIUM OXIDE
0 P's
Absolute
3.5
3.0
2.5
2.0
1.5
1.0
0.5
0
200
180
160
140
120
100
80
60
40
20
0
Chemical Shift (ppm)

Fig. 5

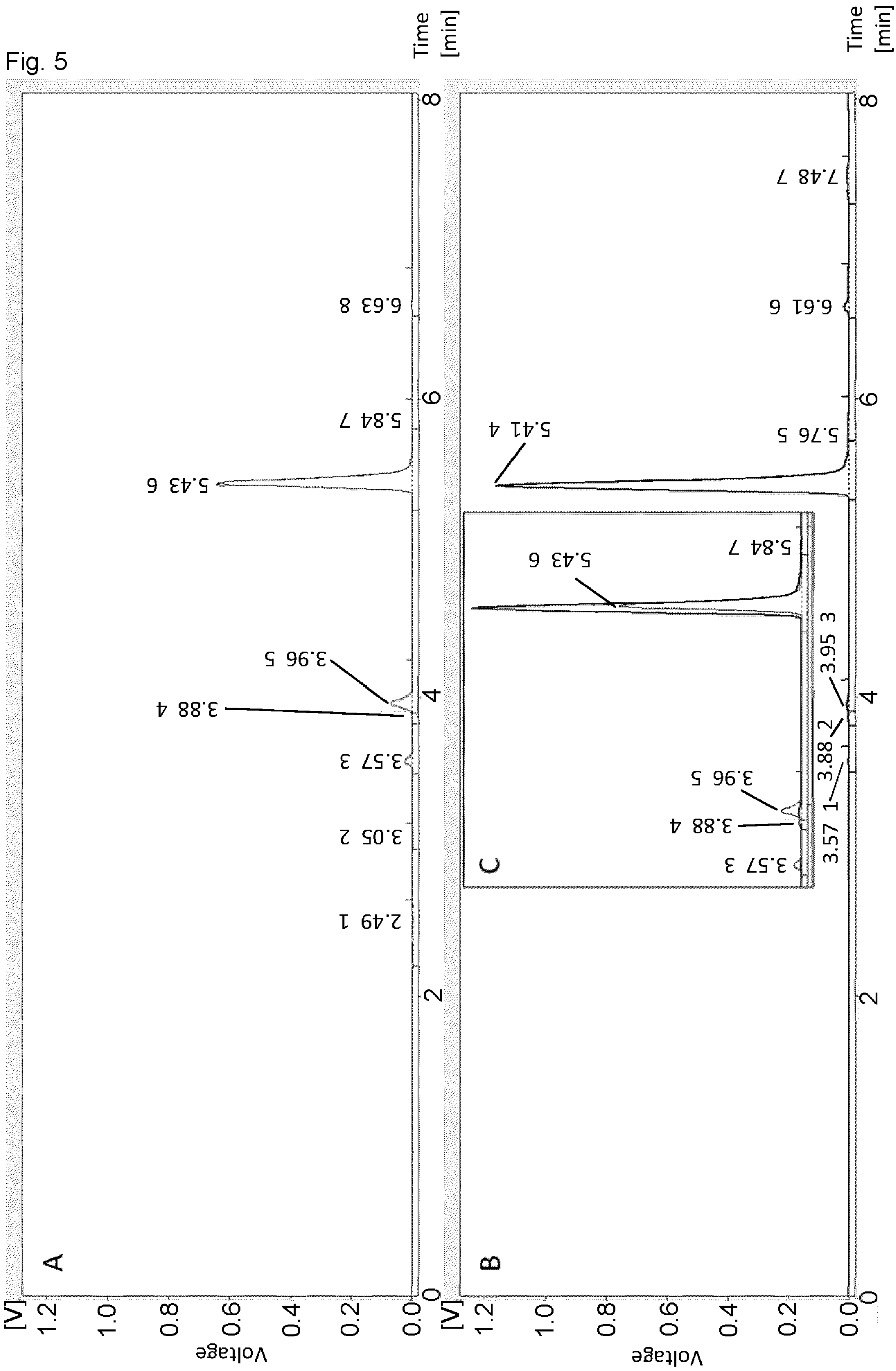
A
B
C
Voltage
[V]
Time [min]
2.49 1
3.05 2
3.57 3
3.88 4
3.96 5
5.43 6
5.84 7
6.63 8
5.41 4
5.76 5
6.61 6
7.48 7
3.57 1
3.88 2
3.95 3

Fig. 6

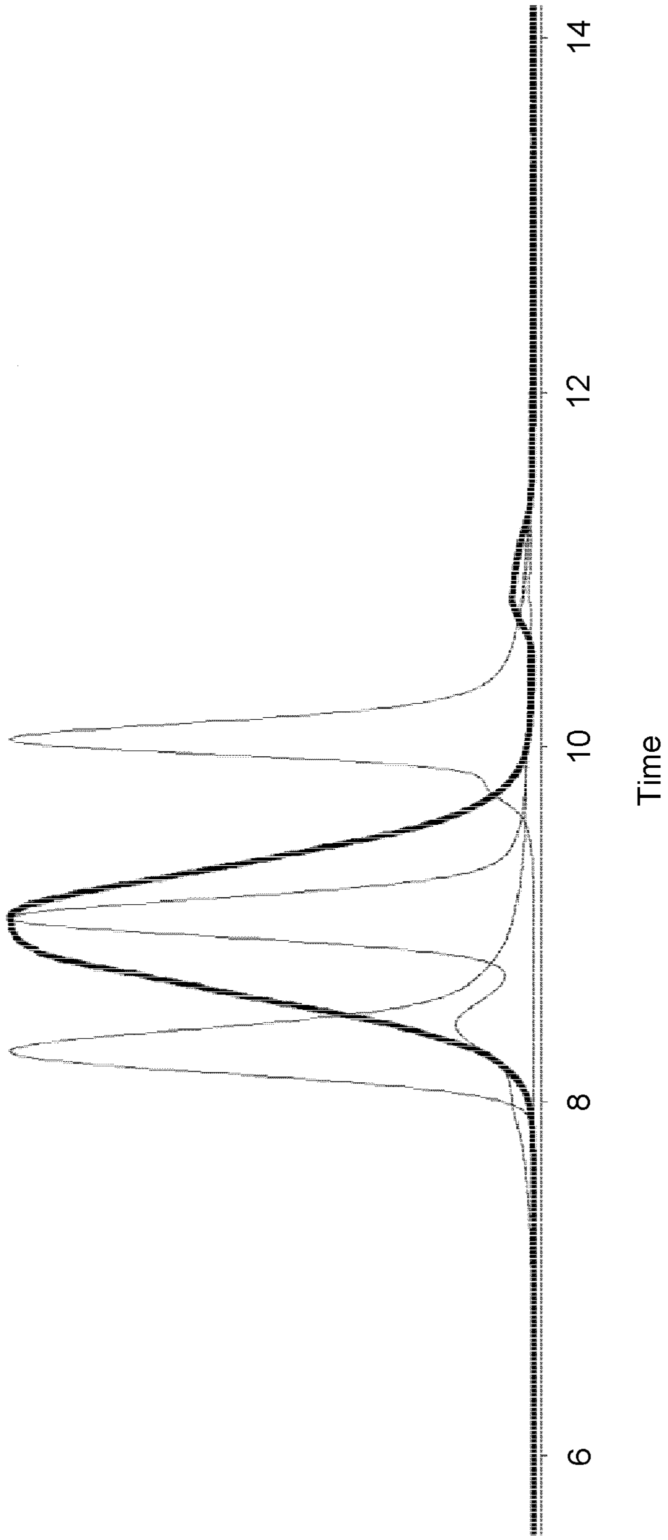

Fig. 7

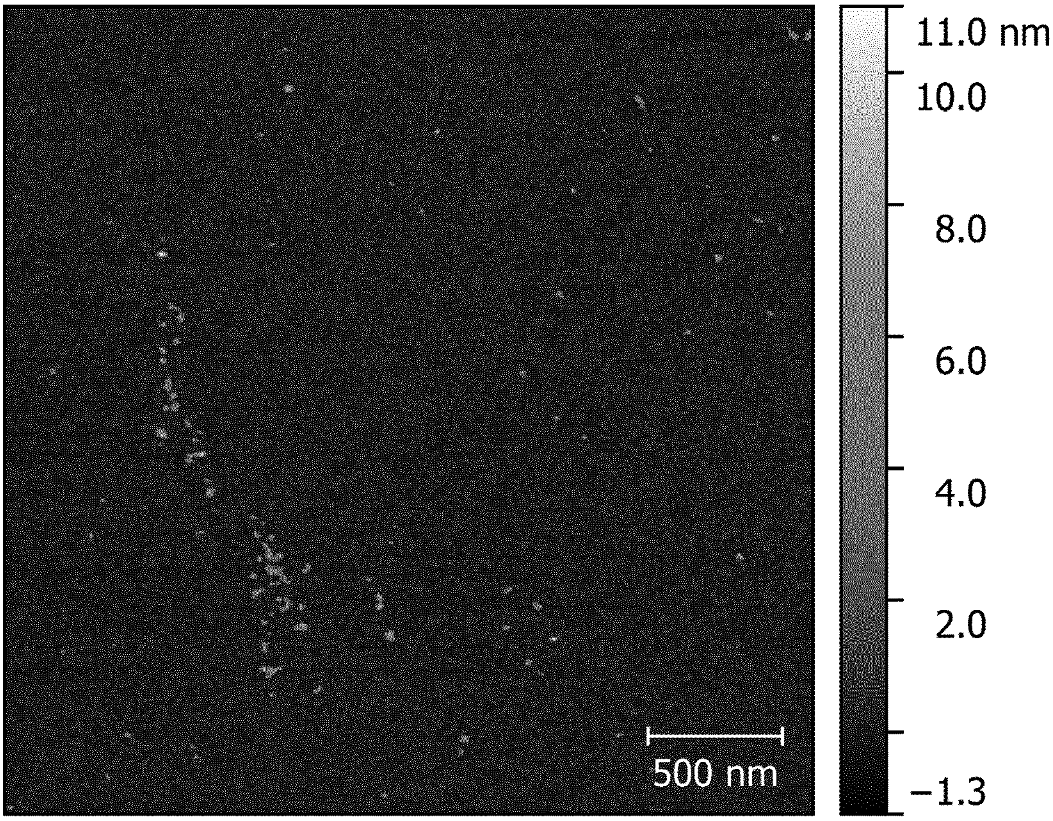

Fig. 8

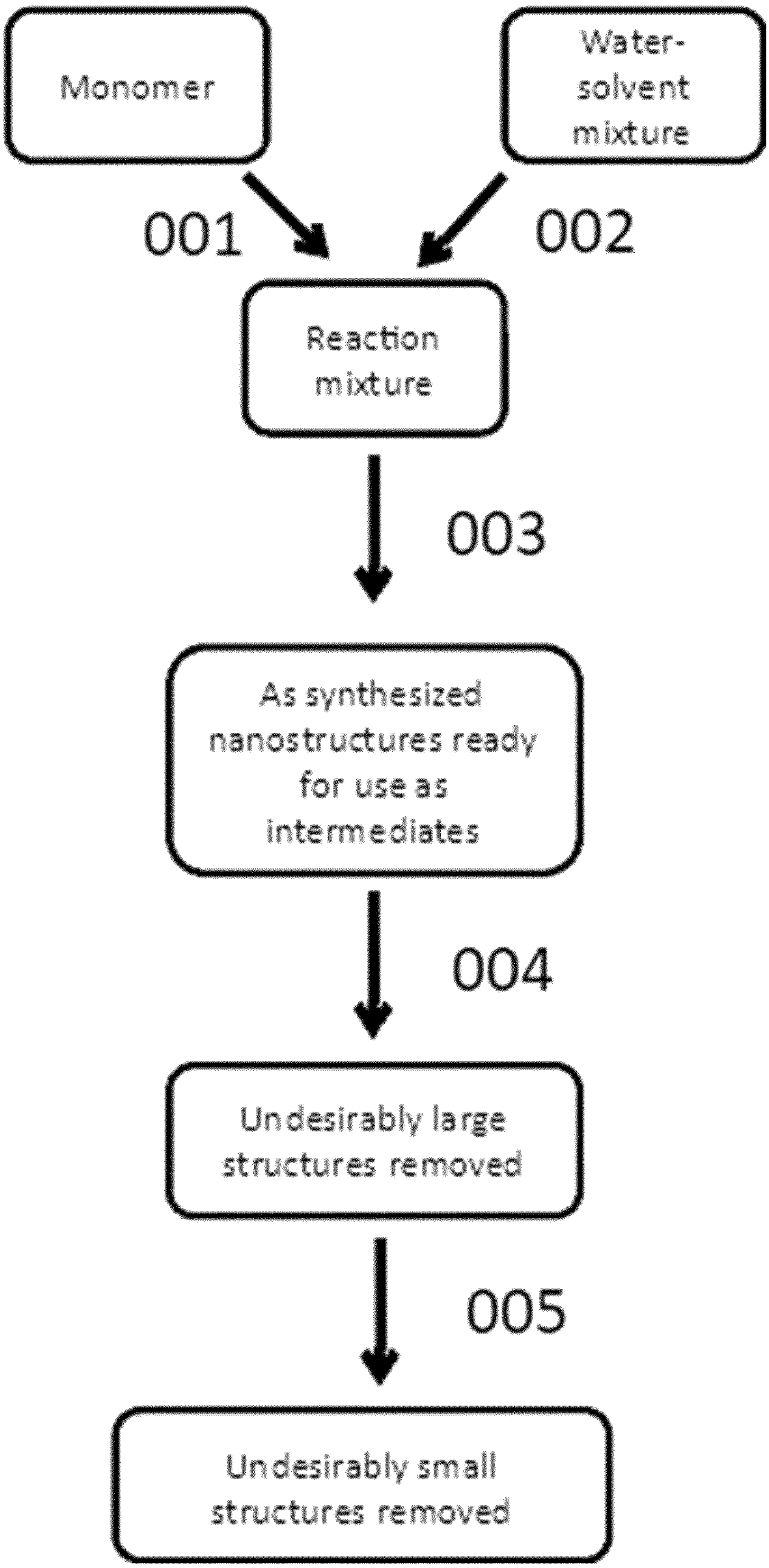
Monomer
Water-solvent mixture
001
002
Reaction mixture
003
As synthesized nanostructures ready for use as intermediates
004
Undesirably large structures removed
005
Undesirably small structures removed

Europäisches Patentamt
European Patent Office
Office européen des brevets

# EUROPEAN SEARCH REPORT

Application Number
EP 22 16 0879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br>Y | WO 2013/041623 A1 (SPAGO IMAGING AB [SE]) 28 March 2013 (2013-03-28)<br>* page 41 - page 42 *<br>----- | 1-8,10, 11,14-16<br>1-16 | INV.<br>A61K49/12<br>A61K49/18 |
| Y | WO 2018/130713 A1 (SPAGO NANOMEDICAL AB [SE]) 19 July 2018 (2018-07-19)<br>* CAS RN 1428320-25-5, 1812171-01-9 *<br>----- | 1-16 | |
| Y | WO 2015/144891 A1 (SPAGO NANOMEDICAL AB [SE]) 1 October 2015 (2015-10-01)<br>* CAS RN 1810844-48-4;<br>page 52 - page 53 *<br>----- | 1-16 | |
| A | GIANOLIO ELIANA ET AL: "Characterization of a Manganese-Containing Nanoparticle as an MRI Contrast Agent",<br>EUROPEAN JOURNAL OF INORGANIC CHEMISTRY,<br>vol. 2019, no. 13,<br>9 April 2019 (2019-04-09), pages 1759-1766, XP055951869,<br>DE<br>ISSN: 1434-1948, DOI: 10.1002/ejic.201801472<br>Retrieved from the Internet:<br>URL:https://onlinelibrary.wiley.com/doi/full-xml/10.1002/ejic.201801472><br>* cas rn 1428320-25-5;<br>figure 1 *<br>----- | 1-16 | TECHNICAL FIELDS SEARCHED (IPC)<br><br>A61K |
| Y | M. DANAEI ET AL: "Impact of Particle Size and Polydispersity Index on the Clinical Applications of Lipidic Nanocarrier Systems",<br>PHARMACEUTICS,<br>vol. 10, no. 2, 18 May 2018 (2018-05-18), page 57, XP055698595,<br>DOI: 10.3390/pharmaceutics10020057<br>* the whole document *<br>-----<br>-/-- | 1-16 | |

The present search report has been drawn up for all claims

1

| Place of search | Date of completion of the search | Examiner |
|---|---|---|
| The Hague | 22 August 2022 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 22 16 0879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KÄTZEL UWE ET AL: "Dynamic Light Scattering for the Characterization of Polydisperse Fractal Systems: II. Relation between Structure and DLS Results", PARTICLE AND PARTICLE SYSTEMS CHARACTERIZATION, vol. 25, no. 1, 1 April 2008 (2008-04-01), pages 19-30, XP055952407, DE ISSN: 0934-0866, DOI: 10.1002/ppsc.200700005 ----- | 1-16 | |
| A | WO 2005/001969 A1 (TORAY INDUSTRIES [JP]; NAKAMURA MASATAKA [JP] ET AL.) 6 January 2005 (2005-01-06) ----- | 1-16 | |
| | | | TECHNICAL FIELDS SEARCHED (IPC) |

The present search report has been drawn up for all claims

1

| Place of search | Date of completion of the search | Examiner |
|---|---|---|
| The Hague | 22 August 2022 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO.

EP 22 16 0879

This annex lists the patent family members relating to the patent documents cited in the above-mentioned European search report. The members are as contained in the European Patent Office EDP file on
The European Patent Office is in no way liable for these particulars which are merely given for the purpose of information.

22-08-2022

| Patent document cited in search report | Publication date | Patent family member(s) | Publication date |
|---|---|---|---|
| WO 2013041623 A1 | 28-03-2013 | AU 2012311522 A1 | 20-03-2014 |
| | | BR 112014006683 A2 | 28-03-2017 |
| | | CA 2847804 A1 | 28-03-2013 |
| | | CN 103930136 A | 16-07-2014 |
| | | DK 2750716 T3 | 30-03-2020 |
| | | EP 2572736 A1 | 27-03-2013 |
| | | EP 2750716 A1 | 09-07-2014 |
| | | ES 2774777 T3 | 22-07-2020 |
| | | IL 231563 A | 29-09-2016 |
| | | JP 6103605 B2 | 29-03-2017 |
| | | JP 2014534277 A | 18-12-2014 |
| | | KR 20140064990 A | 28-05-2014 |
| | | PL 2750716 T3 | 29-06-2020 |
| | | RU 2014111802 A | 27-10-2015 |
| | | US 2014350193 A1 | 27-11-2014 |
| | | WO 2013041623 A1 | 28-03-2013 |
| | | ZA 201402759 B | 29-07-2015 |
| WO 2018130713 A1 | 19-07-2018 | AU 2018208538 A1 | 18-07-2019 |
| | | BR 112019014483 A2 | 11-02-2020 |
| | | CA 3048552 A1 | 19-07-2018 |
| | | CN 110191890 A | 30-08-2019 |
| | | DK 3568403 T3 | 12-07-2021 |
| | | EP 3348560 A1 | 18-07-2018 |
| | | EP 3568403 A1 | 20-11-2019 |
| | | ES 2880228 T3 | 24-11-2021 |
| | | IL 267895 A | 26-09-2019 |
| | | JP 7078274 B2 | 31-05-2022 |
| | | JP 2020507559 A | 12-03-2020 |
| | | KR 20190104598 A | 10-09-2019 |
| | | PL 3568403 T3 | 08-11-2021 |
| | | RU 2019125812 A | 16-02-2021 |
| | | US 2019352459 A1 | 21-11-2019 |
| | | WO 2018130713 A1 | 19-07-2018 |
| WO 2015144891 A1 | 01-10-2015 | AU 2015238208 A1 | 29-09-2016 |
| | | CA 2943852 A1 | 01-10-2015 |
| | | CN 106232146 A | 14-12-2016 |
| | | DK 3122383 T3 | 08-04-2019 |
| | | EP 2923712 A1 | 30-09-2015 |
| | | EP 3122383 A1 | 01-02-2017 |
| | | ES 2715885 T3 | 06-06-2019 |
| | | JP 6707074 B2 | 10-06-2020 |
| | | JP 2017509708 A | 06-04-2017 |
| | | KR 20160137634 A | 30-11-2016 |
| | | PL 3122383 T3 | 30-08-2019 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 22 16 0879

This annex lists the patent family members relating to the patent documents cited in the above-mentioned European search report. The members are as contained in the European Patent Office EDP file on
The European Patent Office is in no way liable for these particulars which are merely given for the purpose of information.

22-08-2022

| Patent document cited in search report | Publication date | Patent family member(s) | Publication date |
|---|---|---|---|
| | | TR 201903815 T4 | 22-04-2019 |
| | | US 2017106105 A1 | 20-04-2017 |
| | | WO 2015144891 A1 | 01-10-2015 |
| WO 2005001969 A1 | 06-01-2005 | CA 2529926 A1 | 06-01-2005 |
| | | CN 1788377 A | 14-06-2006 |
| | | EP 1641063 A1 | 29-03-2006 |
| | | KR 20060036931 A | 02-05-2006 |
| | | US 2007134530 A1 | 14-06-2007 |
| | | WO 2005001969 A1 | 06-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2923712 A1
- US 20140004048 A
- WO 2004040972 A
- WO 2013041623 A1